# EUROPEAN PATENT APPLICATION

(11) **EP 4 491 708 A1**
(43) Date of publication of application: **15.01.2025**
(21) Application number: 22933755.5
(22) Date of filing: 27.07.2022
(51) Int. Cl.: C12N 1/16, C12N 9/10, C12N 15/81, C12P 23/00, C12R 1/645

(54) **MICROORGANISM FOR PRODUCING CAROTENOID OR PRODUCING MATERIAL HAVING CAROTENOID AS PRECURSOR, COMPRISING GERANYLGERANYL PYROPHOSPHATE SYNTHASE DERIVED FROM HAEMATOCOCCUS PLUVIALIS, AND CAROTENOID OR RETINOID PRODUCTION METHOD USING SAME**

(30) Priority: 23.03.2022 KR 20220036258
(71) Applicant: CJ CheilJedang Corporation, Seoul 04560 (KR)
(72) Inventor: LEE, Dong Pil, Seoul 04560 (KR); PARK, Hye Min, Seoul 04560 (KR); LEE, Peter, Seoul 04560 (KR); KIM, Jae Eung, Seoul 04560 (KR)
(74) Representative: Meissner Bolte Partnerschaft mbB
(86) International application number: PCT/KR2022/011049
(87) International publication number: WO 2023/182583

(57) **Abstract**

Provided are a microorganism of the genus *Yarrowia* having an ability to produce carotenoid or a material having carotenoid as a precursor, the microorganism expressing *Haematococcus pluvialis-derived* geranylgeranyl pyrophosphate synthase; a method of producing carotenoid or a material having carotenoid as a precursor using the microorganism; a composition for producing carotenoid or a material having carotenoid as a precursor; and use of the microorganism of the genus *Yarrowia* or a culture thereof in producing carotenoid or a material having carotenoid as a precursor.

## Description

### [Technical Field]

The present disclosure relates to a microorganism of the genus *Yarrowia* having an ability to produce carotenoid or a material having carotenoid as a precursor, the microorganism expressing *Haematococcus pluvialis-derived* geranylgeranyl pyrophosphate synthase; a method of producing carotenoid or a material having carotenoid as a precursor using the microorganism; a composition for producing carotenoid or a material having carotenoid as a precursor; and use of the microorganism of the genus *Yarrowia* or a culture thereof in producing carotenoid or a material having carotenoid as a precursor.

### [Background Art]

As carotenoids and retinoids exert various functions in plants and animals, they are used in various industrial fields such as foods, feeds, etc. Among them, carotenoids such as beta-carotene are substances reported to have functions such as eliminating free radicals, acting as precursors for vitamin A in animals, enhancing the immune system of vertebrates, and reducing the risk of lung cancer, and retinoids are a group of materials chemically related to retinol which is vitamin A, and also used in cosmetics, therapeutic agents for skin diseases, etc.

However, despite these advantages, carotenoids (e.g., beta-carotene) and retinoids (e.g., retinol) are not synthesized in the animal body or are synthesized in insufficient amounts. In addition, even though industrial production is attempted using mutated microorganisms (US Patent No. 7745170), it is still difficult to produce them with high purity.

For example, during preparation of microorganisms producing carotenoids or retinoids, squalene (C30) may also be produced as a by-product. Therefore, the discovery of geranylgeranyl pyrophosphate synthase, which contributes to the efficient production of carotenoids or retinoids, is essential to increasing their production and to reducing squalene which is produced in a competing pathway.

### [Disclosure]

### [Technical Problem]

The problem to be solved in the present disclosure is to provide a microorganism producing carotenoid or a material having carotenoid as a precursor, the microorganism comprising *Haematococcus pluvialis-derived* geranylgeranyl pyrophosphate synthase, a method of producing carotenoid or retinoid using the same and use thereof.

### [Technical Solution]

An object of the present disclosure is to provide a microorganism of the genus *Yarrowia* having an ability to produce carotenoid or a material having carotenoid as a precursor, the microorganism expressing *Haematococcus pluvialis-*derived geranylgeranyl pyrophosphate synthase.

Another object of the present disclosure is to provide a method of producing carotenoid or a material having carotenoid as a precursor using the microorganism of the genus *Yarrowia.*

Still another object of the present disclosure is to provide a composition for producing carotenoid or a material having carotenoid as a precursor, the composition comprising the microorganism of the genus *Yarrowia* or a culture thereof.

Still another object of the present disclosure is to provide use of the microorganism of the genus *Yarrowia* in producing carotenoid or a material having carotenoid as a precursor.

### [Advantageous Effects]

The present disclosure may effectively increase production of carotenoid and a material having carotenoid as a precursor by introducing a *Haematococcus pluvialis-derived* geranylgeranyl pyrophosphate synthase gene into a microorganism of the genus *Yarrowia.*

### [Brief Description of the Drawing]

FIG. 1 shows results of flask tests of strains which were introduced with GGPP synthase genes derived from various microorganisms, respectively; and
FIG. 2 shows results of flask tests of Mb.BCO-introduced strains.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a microorganism of the genus *Yarrowia* having an ability to produce carotenoid or a material having carotenoid as a precursor, the microorganism expressing *Haematococcus pluvialis-derived* geranylgeranyl pyrophosphate synthase.

As used herein, "geranylgeranyl pyrophosphate synthase" is an enzyme capable of catalyzing the synthesis of geranylgeranyl pyrophosphate (GGPP). A substrate of the geranylgeranyl pyrophosphate synthase may be isopentenyl pyrophosphate (IPP) and dimethylallyl pyrophosphate (DMAPP). The geranylgeranyl pyrophosphate synthase may also be named `GGS', 'GGPPS', 'GGPS', `GGPPS1', or 'polypeptide having geranylgeranyl pyrophosphate synthase activity'.

In one embodiment, the microorganism of the present disclosure may be a microorganism of the genus *Yarrowia* comprising or expressing a *Haematococcus pluvialis-derived* geranylgeranyl pyrophosphate synthase protein which is a foreign protein, and may have the ability to produce carotenoid or a material having carotenoid as a precursor.

An amino acid sequence of the GGPPS protein of the present disclosure may be a protein sequence having the geranylgeranyl pyrophosphate synthase activity, which is encoded by the GGPPS gene. The amino acid sequence may be available in various databases, such as the NCBI GenBank, etc., which is a known database, but is not limited thereto.

In one embodiment, the GGPPS protein of the present disclosure may be derived from *Haematococcus pluvialis,* and any protein may be included in the present disclosure as long as it has the sequence or activity identical thereto.

In one embodiment, the GGPPS protein of the present disclosure may comprise, have, or consist of SEQ ID NO: 103 or an amino acid sequence having 80% or more homology or identity thereto, or may essentially consist of the amino acid sequence.

Further, although one embodiment of the GGPPS protein of the present disclosure is described as the protein comprising SEQ ID NO: 103, such expression does not exclude a mutation that may occur by the addition of a meaningless sequence upstream or downstream of the amino acid sequence of SEQ ID NO: 103, or a naturally-occurring mutation therein, or a silent mutation thereof, and it is obvious to those skilled in the art that any protein may fall within the GGPPS protein of the present disclosure, as long as it has activity identical or corresponding to that of the protein comprising the amino acid sequence.

Specifically, the GGPPS protein of the present disclosure may comprise the amino acid sequence of SEQ ID NO: 103 or an amino acid sequence having at least 80%, 85%, 86%, 87%, 88%, 89%, 90%, 91%, 92%, 93%, 94%, 95%, 96%, 97%, 98% or 99% homology or identity to the amino acid sequence of SEQ ID NO: 103. Further, it is apparent that proteins having amino acid sequences in which some sequences are deleted, modified, substituted, or added are also included within the scope of the present disclosure as long as the amino acid sequences have such homology or identity and exhibit the efficacy corresponding to that of the above protein.

Although described as 'a polypeptide or protein comprising an amino acid sequence represented by a particular SEQ ID NO.', 'a polypeptide or protein consisting of an amino acid sequence represented by a particular SEQ ID NO.', or 'a polypeptide or protein having an amino acid sequence represented by a particular SEQ ID NO.' in the present disclosure, it is obvious that a protein having an amino acid sequence with deletion, modification, substitution, conservative substitution, or addition of some sequence may also be used in the present disclosure, as long as it has activity identical or corresponding to that of the polypeptide consisting of the amino acid sequence of the corresponding SEQ ID NO. Examples thereof comprise those having the addition of a sequence that does not alter the function of the protein at the N-terminus, inside, and/or C-terminus of the amino acid sequence, a naturally occurring mutation, a silent mutation thereof, or a conservative substitution.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of polypeptides.

As used herein, the term 'homology' or 'identity' refers to the degree of similarity between two given amino acid sequences or nucleotide sequences and may be expressed as a percentage. The terms 'homology and identity' may be often used interchangeably.

The sequence homology or identity of conserved polynucleotides or polypeptides may be determined by a standard alignment algorithm, and default gap penalties established by a program to be used may be used together. Substantially, homologous or identical sequences may generally hybridize with each other along the entire sequence or at least about 50%, 60%, 70%, 80% or 90% of the entire length under moderate or highly stringent conditions. It is obvious that the hybridization also comprises hybridization with a polynucleotide containing the usual codons or codons considering codon degeneracy in the polynucleotide.

Whether any two polynucleotide or polypeptide sequences have homology, similarity, or identity may be determined using a known computer algorithm such as the "FASTA" program using a default parameter, for example, as in Pearson et al (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, homology, similarity, or identity may be determined using BLAST or ClustalW of the National Center for Biotechnology.

Homology, similarity, or identity of polynucleotides or polypeptides may be determined by comparing sequence information using a GAP computer program, e.g., Needleman et al. (1970), J Mol Biol. 48:443, for example, as disclosed in Smith and Waterman, Adv. Appl. Math (1981) 2:482. Briefly, the GAP program defines similarity as the number of aligned symbols (i.e., nucleotides or amino acids) which are similar, divided by the total number of symbols in the shorter of the two sequences. The default parameters for the GAP program may comprise: (1) a binary comparison matrix (containing a value of 1 for identities and 0 for non-identities) and the weighted comparison matrix (or EDNAFULL (EMBOSS version of NCBI NUC4.4) substitution matrix) of Gribskov et al(1986) Nucl. Acids Res. 14: 6745, as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap open penalty 10, gap extension penalty 0.5); and (3) no penalty for end gaps.

Further, whether any two polynucleotide or polypeptide sequences have homology, similarity, or identity may be determined by comparing these sequences via Southern hybridization experiments under defined stringent conditions, and the appropriate hybridization conditions to be defined may be within the scope of the technology and may be determined by a method well known to one of ordinary skill in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

In the present disclosure, expression of the protein may be achieved by introducing a gene (polynucleotide) encoding the protein into a microorganism or injecting the protein thereto, but is not limited thereto.

In one embodiment, the microorganism of the present disclosure may be introduced with the *Haematococcus pluvialis-derived* geranylgeranyl pyrophosphate synthase gene. Further, introduction of the geranylgeranyl pyrophosphate synthase gene may also additionally include enhancing the activity thereof after the introduction.

As used herein, the 'geranylgeranyl pyrophosphate synthase gene' may be used interchangeably with '*ggs', 'ggpps', 'ggps',* `GGS gene', 'GGPPS gene', 'GGPS gene', 'gene encoding geranylgeranyl pyrophosphate synthase', 'polynucleotide encoding geranylgeranyl pyrophosphate synthase', or 'polynucleotide encoding the polypeptide having the geranylgeranyl pyrophosphate synthase activity'.

The sequence of the geranylgeranyl pyrophosphate synthase gene may be available in various databases, such as the NCBI GenBank, etc., which are known databases, but is not limited thereto.

In one embodiment, the *Haematococcus pluvialis-derived* geranylgeranyl pyrophosphate synthase gene may comprise, have, or consist of a nucleotide sequence of SEQ ID NO: 1, but is not limited thereto.

In one embodiment, the geranylgeranyl pyrophosphate synthase gene consisting of the nucleotide sequence of SEQ ID NO: 1 may be codon-optimized for a microorganism of the genus *Yarrowia,* or more specifically, *Yarrowia lipolytica.*

As used herein, the term "polynucleotide", which is a nucleotide polymer in which nucleotide monomers are covalently bonded in a long chain, refers to a DNA strand having a predetermined length or more.

Further, the polynucleotide or gene may have various modifications in the coding region within a range that does not change the amino acid sequence of the polypeptide, due to codon degeneracy or considering codons preferred by an organism to express the geranylgeranyl pyrophosphate synthase polypeptide. The polynucleotide or gene may comprise, for example, the nucleotide sequence of SEQ ID NO: 1, and may consist of a nucleotide sequence having 80% or more, 90% or more, 95% or more, 96% or more, 97% or more, 98% or more, or 99% or more homology or identity thereto, but is not limited thereto.

Further, the polynucleotide or gene of the present disclosure may comprise a probe that may be prepared from a known gene sequence, for example, any sequence without limitation as long as it is a sequence that hybridizes with a complementary sequence to the entirety or a part of the nucleotide sequence under stringent conditions to encode the amino acid sequence of SEQ ID NO: 103. The "stringent conditions" mean conditions that enable specific hybridization between polynucleotides. These conditions are specifically described in documents (e.g., J. Sambrook et al., supra). For example, the stringent conditions may comprise conditions under which polynucleotides having high homology or identity, for example, 40% or higher, specifically 90% or higher, more specifically 95% or higher, 96% or higher, 97% or higher, 98% or higher, much more specifically 99% or higher homology or identity are hybridized with each other and polynucleotides having homology or identity lower than the above homology or identity are not hybridized with each other, or ordinary washing conditions of Southern hybridization, in which washing is performed once, specifically, two to three times at a salt concentration and temperature equivalent to 60°C, 1X SSC, 0.1% SDS, specifically 60°C, 0.1X SSC, 0.1% SDS, more specifically, 68°C, 0.1X SSC, 0.1% SDS.

Hybridization requires that two nucleic acids have complementary sequences, although mismatches between nucleotides may be possible depending on the stringency of the hybridization. The term "complementary" is used to describe the relationship between mutually hybridizable nucleotides. For example, with respect to DNA, adenosine is complementary to thymine, and cytosine is complementary to guanine. Therefore, the polynucleotide of the present disclosure may also comprise an isolated nucleic acid fragment complementary to the entire sequence as well as a nucleic acid sequence substantially similar thereto.

Specifically, a polynucleotide having homology or identity may be detected using hybridization conditions comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (see Sambrook et al., supra, 9.50-9.51, 11.7-11.8).

In one embodiment, the microorganism of the present disclosure may comprise a vector comprising the *Haematococcus pluvialis-*derived geranylgeranyl pyrophosphate synthase gene of the present disclosure or the polynucleotide encoding the *Haematococcus pluvialis-*derived geranylgeranyl pyrophosphate synthase.

The vector of the present disclosure may comprise a DNA construct comprising a nucleotide sequence of a polynucleotide encoding a polypeptide of interest that is operably linked to a suitable expression regulatory region (or expression control sequence) so that the polypeptide of interest may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDC system, pBR system, pUC system, pBluescript II system, pGEM system, pTZ system, pCL system, pET system, etc. may be used as a plasmid vector. Specifically, pDZ, pDC, pDCM2 (Korean Patent Publication No. 10-2020-0136813), pACYC177, pACYC184, pCL, pECCG117, pUC19, pBR322, pMW118, pCC1BAC, pIMR53 vector, etc. may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and/or RNA encoding a polypeptide of interest. The polynucleotide may be introduced in any form as long as it may be introduced into a host cell and expressed. For example, the polynucleotide may be introduced into a host cell in the form of an expression cassette, which is a gene construct containing all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the desired polypeptide of the present disclosure.

In one embodiment, the microorganism of the genus *Yarrowia* expressing the *Haematococcus pluvialis-derived* GGPPS of the present disclosure may have enhanced geranylgeranyl pyrophosphate synthase activity, as compared to a microorganism of the genus *Yarrowia* not expressing the same, but is not limited thereto.

In one embodiment, the microorganism of the genus *Yarrowia,* into which the *Haematococcus pluvialis-derived* GGPPS gene of the present disclosure is introduced, may have enhanced geranylgeranyl pyrophosphate synthase activity, as compared to a microorganism of the genus *Yarrowia,* into which the *Haematococcus pluvialis-*derived GGPPS gene is not introduced, but is not limited thereto.

In one embodiment, the microorganism of the genus *Yarrowia,* into which the geranylgeranyl pyrophosphate synthase encoded by the *Haematococcus pluvialis-*derived GGPPS gene of the present disclosure is introduced, may have enhanced geranylgeranyl pyrophosphate synthase activity, as compared to a microorganism of the genus *Yarrowia,* into which geranylgeranyl pyrophosphate synthase encoded by *Xanthophyllomyces dendrorhous-*derived crtE or its variant gene *crtEM1, Saccharomyces cerevisiae-*derived BTS1 gene, or *Yarrowia lipolytica-*derived GGS1 gene is introduced, but is not limited thereto.

As used herein, the term "microorganism of the genus *Yarrowia"* or "strain of the genus *Yarrowia"* comprises all of wild-type microorganisms of the genus *Yarrowia* or naturally or artificially genetically modified microorganisms of the genus *Yarrowia,* and it may be a microorganism of the genus *Yarrowia* in which a specific mechanism is strengthened due to insertion of a foreign gene or an activity enhancement of an endogenous gene, and it may be a microorganism of the genus *Yarrowia* comprising the *Haematococcus pluvialis-*derived GGPPS gene, for producing carotenoid or a material having carotenoid as a precursor.

The microorganism of the present disclosure may be a microorganism comprising any one or more of the GGPPS protein of the present disclosure, the GGPS gene or polynucleotide encoding the GGPPS protein, and the vector comprising the gene or polynucleotide; a microorganism modified to express the *Haematococcus pluvialis-*derived GGPPS protein or the GGPPS gene of the present disclosure; a microorganism (e.g., a recombinant strain) expressing the *Haematococcus pluvialis-*derived GGPPS protein or GGPPS gene of the present disclosure; or a strain (e.g., a recombinant strain) having the activity of the *Haematococcus pluvialis*-derived GGPPS of the present disclosure, but is not limited thereto.

The strain of the present disclosure may be a microorganism naturally having the geranylgeranyl pyrophosphate synthase or the ability to produce carotenoid or a material having carotenoid as a precursor; or a microorganism in which the geranylgeranyl pyrophosphate synthase or the ability to produce carotenoid or a material having carotenoid as a precursor is enhanced or provided by introducing the *Haematococcus pluvialis-*derived GGPPS protein, gene, polynucleotide, or the vector comprising the same of the present disclosure into a parent strain not having the geranylgeranyl pyrophosphate synthase or the ability to produce carotenoid or the material having carotenoid as a precursor, but is not limited thereto.

For example, the strain of the present disclosure may comprise all of microorganisms which are transformed with the *Haematococcus pluvialis-*derived GGPPS protein, gene, polynucleotide of the present disclosure, or the vector comprising the same to produce carotenoid or a material having carotenoid as a precursor or to have the enhanced production ability. For example, the strain of the present disclosure may be a recombinant strain having the enhanced ability to produce carotenoid or a material having carotenoid as a precursor by expressing the *Haematococcus pluvialis*-derived GGPPS of the present disclosure in the natural wild-type microorganism or the microorganism producing carotenoid or a material having carotenoid as a precursor. The recombinant strain having the enhanced ability to produce carotenoid or a material having carotenoid as a precursor may be a microorganism having the enhanced ability to produce carotenoid or a material having carotenoid as a precursor, as compared to a natural wild-type microorganism or a geranylgeranyl pyrophosphate synthase-unmodified microorganism (i.e., a microorganism of the genus *Yarrowia* comprising the wild-type geranylgeranyl pyrophosphate synthase gene (SEQ ID NO: 11) or a microorganism of the genus *Yarrowia* into which the *Haematococcus pluvialis-*derived geranylgeranyl pyrophosphate synthase gene (SEQ ID NO: 1) is not introduced, but is not limited thereto.

For example, the strain having the enhanced ability to produce carotenoid or a material having carotenoid as a precursor of the present disclosure may be a microorganism having the enhanced ability to produce carotenoid or a material having carotenoid as a precursor, as compared to a microorganism of the genus Yarrowia comprising no *Haematococcus pluvialis-*derived GGPPS (e.g., SEQ ID NO: 103); or comprising *Xanthophyllomyces dendrorhous-*derived CrtE or its variant CrtEM1, *Saccharomyces cerevisiae-*derived BTS1, or *Yarrowia lipolytica* GGS1, but is not limited thereto. For example, the unmodified microorganism, which is the target strain for comparing whether the ability to produce carotenoid or a material having carotenoid as a precursor is enhanced or not, may be a strain CC08-1023, but is not limited thereto.

For example, the recombinant strain having the enhanced production ability may have about 0.001% or more or 0.01% or more enhancement of the beta-carotene or retinol-producing ability, as compared to that of the parent strain before modification or the unmodified microorganism. However, as long as the microorganism has an increased ability of + value, as compared to that of the parent strain before modification or the unmodified microorganism, it is not limited thereto. The term "about" refers to a range which includes all of ±0.5, ±0.4, ±0.3, ±0.2, ±0.1, etc., and includes all of the values that are equivalent or similar to those following the term "about", but the range is not limited thereto.

As used herein, the term "unmodified microorganism" does not exclude strains comprising mutations that may occur naturally in microorganisms, and may be a wild-type strain or a natural strain itself or may be a strain before the trait is changed by genetic variation due to natural or artificial factors. For example, the unmodified microorganism may be a strain in which the *Haematococcus pluvialis-*derived GGPPS is not expressed, or into which the *Haematococcus pluvialis-*derived GGPPS has not yet been introduced. The term "unmodified microorganism" may be used interchangeably with "strain before being modified", "microorganism before being modified", "unvaried strain", "unmodified strain", "unvaried microorganism", or "reference microorganism".

The microorganism of the present disclosure may be a microorganism of the genus *Yarrowia,* specifically, *Yarrowia lipolytica,* but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation, and/or chemicals, but is not limited thereto. A method of modifying a part or the entirety of the gene may comprise a method of using a DNA recombination technology. For example, by introducing a nucleotide sequence or vector comprising a nucleotide sequence homologous to the gene of interest into the microorganism to cause homologous recombination, a part or the entirety of the gene may be deleted. The nucleotide sequence or vector to be introduced may comprise a dominant selection marker, but is not limited thereto.

The microorganism of the present disclosure may be a microorganism of the genus *Yarrowia* that is modified to comprise polynucleotides encoding lycopene cyclase/phytoene synthase (crtYB), phytoene desaturase (crtl), and beta-carotene 15,15'-oxygenase (BLH) proteins.

The microorganism of the present disclosure may be a microorganism that is modified to further comprise polynucleotides encoding lycopene cyclase/phytoene synthase (crtYB) and phytoene desaturase (crtl) proteins, thereby exhibiting activities of the proteins, or a microorganism in which activities of the proteins are enhanced. The lycopene cyclase/phytoene synthase or phytoene desaturase may be a protein derived from *Xanthophyllomyces dendrorhous,* but is not limited thereto. In one embodiment, the polynucleotide encoding the lycopene cyclase/phytoene synthase or phytoene desaturase may have or may comprise a sequence, based on a nucleotide sequence (GenBank: AY177204.1 or GenBank: AY177424.1) registered in the National Center for Biotechnology Information Search database (NCBI), respectively. In one embodiment, the polynucleotide encoding the lycopene cyclase/phytoene synthase or phytoene desaturase may have or comprise SEQ ID NO: 71 or SEQ ID NO: 72, respectively. In the polynucleotide, various modifications may be made in the coding region as long as the amino acid sequence is not changed in consideration of codon degeneracy or codons preferred in microorganisms that are intended to express the polypeptide of the present disclosure. Specifically, the polynucleotide may have or may comprise a nucleotide sequence having 80% or more, 85% or more, 90% or more, 95% or more, 96% or more, 97% or more, 98% or more, and less than 100% homology or identity to the sequence of SEQ ID NO: 71 or SEQ ID NO: 72, or may consist of or may essentially consist of a nucleotide sequence having 80% or more, 85% or more, 90% or more, 95% or more, 96% or more, 97% or more, 98% or more, and less than 100% homology or identity to the sequence of SEQ ID NO: 71 or SEQ ID NO: 72, but is not limited thereto.

The microorganism of the present disclosure may be a microorganism that is modified to further comprise a polynucleotide encoding beta-carotene 15,15'-oxygenase (BLH) protein, thereby exhibiting activity of the protein, or a microorganism in which the activity of the protein is enhanced, but is not limited thereto. The beta-carotene 15, 15'-oxygenase may be a protein derived from *Uncultured marine bacterium* 66A03, but is not limited thereto. In one embodiment, the polynucleotide encoding beta-carotene 15, 15'-oxygenase may have or comprise an amino acid sequence, based on Q4PNI0 which is registered in UniProt Knowledgebase (UniProtKB). In one embodiment, the polynucleotide encoding beta-carotene 15, 15'-oxygenase may have or comprise a sequence of SEQ ID NO: 13. The polynucleotide may undergo various modifications in the coding region within the scope that does not change the amino acid sequence in consideration of codon degeneracy or codons preferred in microorganisms that are intended to express the polypeptide of the present disclosure. Specifically, the polynucleotide may have or comprise a nucleotide sequence having 80% or more, 85% or more, 90% or more, 95% or more, 96% or more, 97% or more, 98% or more, and less than 100% homology or identity to the sequence of SEQ ID NO: 13, or may consist of or essentially consist of a nucleotide sequence having 80% or more, 85% or more, 90% or more, 95% or more, 96% or more, 97% or more, 98% or more, and less than 100% homology or identity to the sequence of SEQ ID NO: 13, but is not limited thereto.

As used herein, the term "enhancement" of polypeptide activity means that the activity of a polypeptide is increased, as compared to the endogenous activity. The enhancement may be used interchangeably with terms such as activation, up-regulation, overexpression, and increase, etc. Here, activation, enhancement, up-regulation, overexpression, and increase may comprise both exhibiting activity that was not originally possessed and exhibiting improved activity as compared to the endogenous activity or activity before modification. The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of the polypeptide is improved as compared to the activity and/or concentration (expression level) of a specific polypeptide originally possessed by a parent strain before the trait is changed or an unmodified microorganism.

The enhancement may be achieved through the introduction of a foreign polypeptide or gene or the enhancement of endogenous activity and/or concentration (expression level) of the polypeptide. The enhancement of the activity of a polypeptide may be confirmed by an increase in the degree of activity and the expression level of the corresponding polypeptide or in the amount of the product produced from the corresponding polypeptide.

For the enhancement of the activity of the polypeptide, various methods well known in the art may be applied, and the method is not limited as long as the activity of the polypeptide of interest may be enhanced as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a nucleotide sequence encoding a start codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically,
1) The increase in the intracellular copy number of the polynucleotide encoding the polypeptide may be achieved by the introduction of, into a host cell, a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into a chromosome of a host cell. The introduction into the chromosome may be performed by introducing a vector capable of inserting the polynucleotide into a chromosome of a host cell into the host cell, but is not limited thereto. The vector is as described above.
2) The replacement of a gene expression regulatory region (or expression control sequence) on a chromosome encoding the polypeptide with a sequence exhibiting strong activity may be, for example, the occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may comprise, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.
   Examples of known strong promoters comprise CJ1 to CJ7 promoters (US Patent No. US 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. US 10584338 B2), O2 promoter (US Patent No. US 10273491 B2), tkt promoter, yccA promoter, TEFINt promoter, etc., but is not limited thereto.
3) The modification of a nucleotide sequence encoding a start codon or 5'-UTR region of the gene transcript encoding the polypeptide may be, for example, substitution with a nucleotide sequence encoding another start codon having a higher polypeptide expression rate as compared to an endogenous start codon, but is not limited thereto.
4) and 5) The modification of the amino acid sequence or the polynucleotide sequence may be the occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further comprise a selection marker for the confirmation of chromosome insertion. The selection marker is as described above.
6) The introduction of a foreign polynucleotide exhibiting the activity of the polypeptide may be the introduction of a foreign polynucleotide encoding a polypeptide exhibiting activity identical or similar to that of the polypeptide into a host cell. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in a host cell, the polypeptide may be produced, and the activity thereof may be increased.
7) The codon optimization of the polynucleotide encoding the polypeptide may be the codon optimization of an endogenous polynucleotide so as to increase transcription or translation in a host cell, or the codon optimization of a foreign polynucleotide so as to perform optimized transcription and translation in a host cell.
8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration expression level of the corresponding polypeptide, based on the activity or concentration of the polypeptide expressed in a wild-type or a microbial strain before being modified, or an increase in the amount of a product produced from the corresponding polypeptide, but is not limited thereto.

In one embodiment, the microorganism of the present disclosure may have the enhanced GGPPS activity by introducing the *Haematococcus pluvialis-derived* GGPPS gene, but is not limited thereto.

The microorganism of the present disclosure may have the ability to produce carotenoid or a material having carotenoid as a precursor.

As used herein, the term "carotenoid" refers to tetraterpene or a derivative thereof that gives colors such as yellow in fruits and vegetables.

In one embodiment, the carotenoid may be any one or more selected from the group consisting of xanthophyll, carotene, alpha-carotene, beta-carotene, gamma-carotene, phytoene, phytofluene, neurosporene, lutein, lycopene, zeaxanthin, capsanthin, canthaxanthin, and astaxanthin, but is not limited thereto.

In one embodiment, the material having carotenoid as a precursor may be retinoid, but is not limited thereto.

As used herein, the term "retinoid" chemically refers to the vitamin A group or a group of compounds chemically related thereto.

In one embodiment, the retinoid may be any one selected from the group consisting of retinol, retinal, retinoic acid, and retinyl ester, but is not limited thereto.

In one embodiment, the microorganism of the present disclosure may have a reduced ability to produce a by-product, but is not limited thereto.

In the present disclosure, the by-product may refer to any material other than carotenoid or the material having carotenoid as a precursor during production thereof. For example, a representative by-product generated during beta-carotene production may be squalene.

As used herein, the "squalene" is an unsaturated hydrocarbon (C₃₀H₅₀) and is a material that is also used in the biosynthesis of steroid hormones, vitamin D, etc. The microorganism of the present disclosure may have the reduced byproducts which are generated in the beta-carotene production pathway, and specifically, may have the reduced squalene production, but is not limited thereto.

Another aspect of the present disclosure provides a method of producing carotenoid or the material having carotenoid as a precursor, the method comprising the step of culturing, in a medium, the microorganism of the genus *Yarrowia* of the present disclosure.

The microorganism, carotenoid, and material having carotenoid as a precursor are as described in other aspects.

As used herein, the term "culturing" refers to growing the microorganism of the genus *Yarrowia* of the present disclosure in appropriately adjusted environmental conditions. In the present disclosure, the culturing procedure may be performed according to appropriate media or culture conditions known in the art. Such culturing procedure may be easily adjusted according to the selected microorganism by a person skilled in the art. Specifically, the culturing may be in a batch type, a continuous type, and/or a fed-batch type, but is not limited thereto.

The microorganism of the genus *Yarrowia* of the present disclosure may be cultured under aerobic conditions in a common medium containing appropriate carbon sources, nitrogen sources, phosphorus sources, inorganic compounds, amino acids, and/or vitamins, while controlling the temperature, pH, etc.

In the culturing of the present disclosure, the culture temperature may be maintained at 20°C to 35°C, specifically, at 25°C to 35°C, and the culturing may be performed for about 10 hours to about 160 hours, about 20 hours to about 130 hours, about 24 hours to about 120 hours, about 36 hours to about 120 hours, about 48 hours to about 120 hours, about 48 hours, about 72 hours, or about 120 hours, but is not limited thereto.

The carotenoid or the material having carotenoid as a precursor which is produced by the culturing of the present disclosure may be released into the medium or may remain in microorganisms.

The method of producing carotenoid or the material having carotenoid as a precursor of the present disclosure may further comprise the steps of preparing the microorganism of the genus *Yarrowia* of the present disclosure, preparing a medium for culturing the microorganism, or a combination of these steps (regardless of the order, in any order), for example, before or after the culturing step.

The method of producing carotenoid or the material having carotenoid as a precursor of the present disclosure may further comprise the step of recovering carotenoid or the material having carotenoid as a precursor from the medium resulting from the culturing of the microorganism of the genus *Yarrowia* (medium in which culturing has been performed) or from the microorganism of the genus *Yarrowia* of the present disclosure. The recovering step may be further included after the culturing step.

The recovering may be collecting the desired retinol by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, cell disruption, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and retinol may be recovered from the medium or microorganism by using an appropriate method known in the art.

In addition, the method of producing carotenoid or the material having carotenoid as a precursor of the present disclosure may further comprise a purification step. The purification may be performed by using an appropriate method known in the art. In an exemplary embodiment, when the method of producing carotenoid or the material having carotenoid as a precursor of the present disclosure comprises both the recovering step and the purification step, the recovering step and the purification step may be performed discontinuously (or continuously) regardless of the order, or may be performed simultaneously or integrated into one step, but is not limited thereto.

The method of producing carotenoid of the present disclosure may further comprise the step of converting beta-carotene, which is produced by the microorganism of the present disclosure, into carotenoids other than beta-carotene. In the method of producing carotenoids of the present disclosure, the converting step may be further included after the culturing step or the recovering step. The converting step may be performed using an appropriate method known in the art. For example, the converting may be performed chemically or using an enzyme, but is not limited thereto.

The method of producing retinoid of the present disclosure may further comprise the step of converting retinol, which is produced by the microorganism of the genus *Yarrowia* of the present disclosure, into retinoids other than retinol. In the method of producing retinoids of the present disclosure, the converting step may be further included after the culturing step or the recovering step. The converting step may be performed using an appropriate method known in the art. For example, the converting may be performed using retinol acyltransferase, but is not limited thereto.

In one embodiment, retinoids other than retinol may be any one selected from the group consisting of retinal, retinoic acid, and retinyl ester, but is not limited thereto, as long as it is included in retinoids.

Still another aspect of the present disclosure provides a composition for producing carotenoid or the material having carotenoid as a precursor, the composition comprising the microorganism of the genus *Yarrowia* of the present disclosure or a culture thereof.

The microorganism, carotenoid, or material having carotenoid as a precursor is as described in other aspects.

The composition of the present disclosure may further include any appropriate excipient commonly used, and the excipient may include, for example, a preserving agent, a wetting agent, a dispersing agent, a suspending agent, a buffer, a stabilizing agent, an isotonic agent, etc., but are not limited thereto.

Still another aspect of the present disclosure provides use of the microorganism of the present disclosure or a culture thereof in producing carotenoid or a material having carotenoid as a precursor.

The microorganism, carotenoid, or material having carotenoid as a precursor are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Preparation of Platform Strains for Producing Carotenoid or Material Having Carotenoid as Precursor

### Example 1-1. Preparation of X. dendrorhous-derived crtYB-crtl inserted strain

To prepare platform strains for producing carotenoid or a material having carotenoid as a precursor, lycopene cyclase/phytoene synthase (crtYB) and phytoene desaturase (crtl) genes derived from *Xanthophyllomyces dendrorhous* were inserted into the genome of a high-fat yeast *Yarrowia lipolytica* CC08-0125 (Accession No. KCCM12972P) strain.

With regard to crtYB, a polynucleotide of SEQ ID NO: 71 was obtained, based on a nucleotide sequence (GenBank: AY177204.1) registered in the National Center for Biotechnology Information Search database (NCBI), and with regard to crtl, a polynucleotide of SEQ ID NO: 72 was obtained, based on a nucleotide sequence (GenBank: AY177424.1) registered in the NCBI. The polynucleotide sequences of crtYB and crtl were synthesized by Macrogen in the form of TEFINtp-crtYB-CYC1t (SEQ ID NO: 73), and TEFINtp-crtl-CYC1t (SEQ ID NO: 74), respectively. A cassette to be inserted into the MHY1(YALI0B21582g) gene site was designed using a URA3 gene (SEQ ID NO: 75) of Y. *lipolytica* as a selection marker. Each PCR was performed using the synthesized crtYB and crtl genes and KCCM12972P genomic DNA as templates, and primers of SEQ ID NO: 76 and SEQ ID NO: 77, SEQ ID NO: 78 and SEQ ID NO: 79, SEQ ID NO: 80 and SEQ ID NO: 81, SEQ ID NO: 82 and SEQ ID NO: 83, SEQ ID NO: 84 and SEQ ID NO: 85, and SEQ ID NO: 86 and SEQ ID NO: 87. PCR was performed by 35 cycles consisting of denaturation at 95°C for 1 min; annealing at 55°C for 1 min; and polymerization reaction at 72°C for 3 min. The resulting DNA fragments were prepared as a single cassette through overlap extension PCR.

The cassette thus prepared was introduced into KCCM12972P strain by a heat shock method (D.-C. Chen et al., Appl Microbiol Biotechnol, 1997), and then colonies were obtained, which were formed on a solid medium (YLMM1) without uracil. Colonies in which cassette insertion into the genome was confirmed using primers of SEQ ID NO: 88 and SEQ ID NO: 89 were spotted on a 5-FOA solid medium and cultured at 30°C for 3 days, and colonies grown on the 5-FOA solid medium were obtained to recover the URA3 marker.

**[Table 1]**

| SEQ ID NO. | Sequence (5'-3') | PCR product |
|---|---|---|
| 76 | GTGCGCTTCTCTCGTCTCGGTAACCCTGTC | Homology left arm |
| 77 | ATGCGCCGCCAACCCGGTCTCTGGGGTGTGGTGGATGGGGTGTG | |
| 78 | CACACCCCATCCACCACACCCCAGAGACCGGGTTGGCGGCGCAT | TEFINtp-crtYB-CYC1t |
| 79 | CGCCGCCAACCCGGTCTCTTGAAGACGAAAGGGCCTCCG | |
| 80 | CGGAGGCCCTTTCGTCTTCAAGAGACCGGGTTGGCGGCG | TEFINtp-crtl- |
| 81 | GACGAGTCAGACAGGAGGCATCAGACAGATACTCGTCGCG | CYC1t |
| 82 | CGCGACGAGTATCTGTCTGATGCCTCCTGTCTGACTCGTC | URA3 |
| 83 | ATGACGAGTCAGACAGGAGGCATGGTGGTATTGTGACTGGGGAT | |
| 84 | ATCCCCAGTCACAATACCACCATGCCTCCTGTCTGACTCGTCAT | Repeat region |
| 85 | CGGCGTCCTTCTCGTAGTCCGCTTTTGGTGGTGAAGAGGAGACT | |
| 86 | AGTCTCCTCTTCACCACCAAAAGCGGACTACGAGAAGGACGCCG | Homology right arm |
| 87 | CCACTCGTCACCAACAGTGCCGTGTGTTGC | |
| 88 | TCGTACGTCTATACCAACAGATGG | Forward |
| 89 | CGCATACACACACACTGCCGGGGG | Reverse |

### Example 1-2. Preparation of HMGR-enhanced strain

A cassette for replacement of a native promoter (SEQ ID NO: 90) region of 3-hydroxy-3-methylglutaryl-CoA reductase (HMGR) gene of the strain which was prepared through Example 1-1 with a TEFINt promoter was designed, and each PCR was performed using genomic DNA of KCCM12972P as a template, and primers of SEQ ID NO: 91 and SEQ ID NO: 92, SEQ ID NO: 93 and SEQ ID NO: 94, SEQ ID NO: 95 and SEQ ID NO: 96, SEQ ID NO: 97 and SEQ ID NO: 98, and SEQ ID NO: 99 and SEQ ID NO: 100. PCR was performed by 35 cycles consisting of denaturation at 95°C for 1 min; annealing at 55°C for 1 min; and polymerization reaction at 72°C for 1 min and 30 sec. The resulting five DNA fragments were prepared as a single cassette through overlap extension PCR.

The cassette thus prepared was introduced into the strain prepared in Example 1-1 by a heat shock method, and then colonies were obtained, which were formed on a solid medium (YLMM1) without uracil. Colonies in which cassette insertion was confirmed using primers of SEQ ID NO: 101 and SEQ ID NO: 102 were spotted on a 5-FOA solid medium and cultured at 30°C for 3 days, and colonies grown on the 5-FOA solid medium were obtained to recover the URA3 marker. Thus, the platform strain finally prepared was named CC08-1023.

### <Yarrowia lipolytica minimal media1 (YLMM1)>

20 g/L of glucose, 6.7 g/L of yeast nitrogen base without amino acids, 2 g/L of yeast synthetic drop-out medium supplements without uracil, 15 g/L of agar

### <5-Fluoroorotic Acid (5-FOA)>

20 g/L of glucose, 6.7 g/L of yeast nitrogen base without amino acids, 2 g/L of yeast synthetic drop-out medium supplements without uracil, 50 µg/mL of uracil, 1 g/L of 5-fluoroorotic acid (5-FOA), 15 g/L of agar

**[Table 2]**

| SEQ ID NO. | Sequence (5'-3') | PCR product |
|---|---|---|
| 91 | GACAATGCCTCGAGGAGGTTTAAAAGTAACT | Homology left arm |
| 92 | GCGCCGCCAACCCGGTCTCTCTGTGTTAGTCGGATGATAGG | |
| 93 | CCTATCATCCGACTAACACAGAGAGACCGGGTTGGCGGCGC | TEFINt promoter |
| 94 | GACGAGTCAGACAGGAGGCACTGCGGTTAGTACTGCAAAAAG | |
| 95 | CTTTTTGCAGTACTAACCGCAGTGCCTCCTGTCTGACTCGTC | URA3 |
| 96 | ATGCGCCGCCAACCCGGTCTCTTGGTGGTATTGTGACTGGGGAT | |
| 97 | ATCCCCAGTCACAATACCACCAAGAGACCGGGTTGGCGGCGCAT | Repeat region |
| 98 | CTTTCCAATAGCTGCTTGTAGCTGCGGTTAGTACTGCAAAA | |
| 99 | TTTTGCAGTACTAACCGCAGCTACAAGCAGCTATTGGAAAG | Homology right arm |
| 100 | GCTTAATGTGATTGATCTCAAACTTGATAG | |
| 101 | GCTGTCTCTGCGAGAGCACGTCGA | Forward |
| 102 | GGTTCGCACAACTTCTCGGGTGGC | Reverse |

### Example 2. Preparation of Haematococcus pluvialis-Derived Geranylgeranyl Pyrophosphate Synthase (GGPP synthase) Gene-Inserted Strain

Four types of GGPP synthase genes (hereinafter, referred to as GGPPS genes) derived from different origins were introduced into the genome of the strain CC08-1023 prepared in Example 1 as follows.

### Example 2-1. Preparation of Haematococcus pluvialis-derived GGPPS-inserted strain

To insert the *Haematococcus pluvialis-*derived GGPPS1 gene (hereinbelow, referred to as Hp.GGPPS1) into the chromosome of *Yarrowia lipolytica,* codon optimization (SEQ ID NO: 1) of Hp.GGPPS1 was performed to be suitable for Y. *lipolytica* through http://atgme.org, based on a nucleotide sequence (GenBank: APX64485.1) registered in National Center for Biotechnology Information Search database (NCBI), and the gene (SEQ ID NO: 4) was synthesized by Macrogen in the form of TEFINtp-codon optimized Hp.GGPPS1-CYC1t. A cassette to be inserted into the LIG4(YALI0D21384g) gene site was designed using the URA3 gene (SEQ ID NO: 5) of Y. *lipolytica* as a selection marker.

PCR was performed for left homologous region, TEFINt promoter, Hp.GGPPS1 ORF, CYC1 terminator, URA3, repeat region, and right homologous region fragments using the synthesized Hp.GGPPS1 gene and genomic DNA of KCCM12972P as templates, and primers of SEQ ID NO: 15 and SEQ ID NO: 16, SEQ ID NO: 17 and SEQ ID NO: 18, SEQ ID NO: 19 and SEQ ID NO: 20, SEQ ID NO: 21 and SEQ ID NO: 22, SEQ ID NO: 23 and SEQ ID NO: 24, SEQ ID NO: 25 and SEQ ID NO: 26, and SEQ ID NO: 27 and SEQ ID NO: 28, as shown in Table 3, respectively. PCR was performed by 35 cycles consisting of denaturation at 95°C for 1 min; annealing at 55°C for 1 min; and polymerization reaction at 72°C for 2 min. The resulting DNA fragments were prepared as a single cassette through overlap extension PCR.

The cassette thus prepared was introduced into the CC08-1023 strain by a heat shock method, and then colonies were obtained, which were formed on a solid medium (YLMM1) without uracil. Colonies in which cassette insertion into the genome was confirmed using primers of SEQ ID NO: 29 and SEQ ID NO: 30 were plated on a 5-FOA solid medium and cultured at 30°C for 3 days, and colonies grown on the 5-FOA solid medium were obtained to remove the URA3 marker.

**[Table 3]**

| SEQ ID NO. | Sequence (5'-3') |
|---|---|
| 15 | CATCATTTCAAAAGAGGGAACAGC |
| 16 | CGCCGCCAACCCGGTCTCTGTGTTTGGCGGTGTGAGTTGTC |
| 17 | GACAACTCACACCGCCAAACACAGAGACCGGGTTGGCGGCG |
| 18 | CGGTTGTGCATGGCTCGGATCTGCGGTTAGTACTGCAAAAGT GC |
| 19 | GCACTTTTTGCAGTACTAACCGCAGATCCGAGCCATGCACAACC G |
| 20 | AACTAATTACATGActcgaGCTAGTTCTTTCGGTAGCCGA |
| 21 | TCGGCTACCGAAAGAACTAGCtcgagTCATGTAATTAGTT |
| 22 | gacgagtcagacaggaggcaGCAAATTAAAGCCTTCGAGC |
| 23 | GCTCGAAGGCTTTAATTTGCtgcctcctgtctgactcgtc |
| 24 | AACTAATTACATGActcgaGtggtggtattgtgactgggg |
| 25 | ccccagtcacaataccaccaCtcgagTCATGTAATTAGTT |
| 26 | CCATATGGAGTGTTATTTGAAGGGGCAAATTAAAGCCTTCGAGC |
| 27 | GCTCGAAGGCTTTAATTTGCCCCTTCAAATAACACTCCATATGG |
| 28 | CCGATACAGTGTCCAAGTACG |
| 29 | GAGTGTCTGAAGACAAGGCTTC |
| 30 | GACGACAATGCTGAGCTCCG |

### Example 2-2. Preparation of Xanthophyllomyces dendrorhous-derived crtE variant gene-inserted strain

To insert the *Xanthophyllomyces dendrorhous-*derived crtE variant gene crtEM1 (SEQ ID NO: 6, Hong et al., Applied Microbiology and Biotechnology, 2019 Jan;103(1):211-223) into the chromosome of *Yarrowia lipolytica,* the gene (SEQ ID NO: 8) was synthesized by Macrogen in the form of TEFINtp-crtEM1-TDH3t. A cassette to be inserted into the LIG4(YALI0D21384g) gene site was designed using the URA3 gene (SEQ ID NO: 5) of Y. *lipolytica* as a selection marker.

PCR was performed for left homologous region, TEFINt promoter, crtEM1 ORF, TDH3 terminator, URA3, repeat region, and right homologous region fragments using the synthesized crtEM1 DNA and genomic DNA of KCCM12972P as templates, and primers of SEQ ID NO: 31 and SEQ ID NO: 32, SEQ ID NO: 33 and SEQ ID NO: 34, SEQ ID NO: 35 and SEQ ID NO: 36, SEQ ID NO: 37 and SEQ ID NO: 38, SEQ ID NO: 39 and SEQ ID NO: 40, SEQ ID NO: 41 and SEQ ID NO: 42, and SEQ ID NO: 43 and SEQ ID NO: 44, as shown in Table 4, respectively.

PCR was performed by 35 cycles consisting of denaturation at 95°C for 1 min; annealing at 55°C for 1 min; and polymerization reaction at 72°C for 2 min. The resulting DNA fragments were prepared as a single cassette through overlap extension PCR.

The cassette thus prepared was introduced into the CC08-1023 strain by a heat shock method, and then colonies were obtained, which were formed on a solid medium (YLMM1) without uracil. Colonies in which cassette insertion into the genome was confirmed using primers of SEQ ID NO: 45 and SEQ ID NO: 46 were plated on a 5-FOA solid medium and cultured at 30°C for 3 days, and colonies grown on the 5-FOA solid medium were obtained to remove the URA3 marker.

**[Table 4]**

| SEQ ID NO. | Sequence (5'-3') |
|---|---|
| 31 | CATCATTTCAAAAGAGGGAACAGC |
| 32 | CGCCGCCAACCCGGTCTCTGTGTTTGGCGGTGTGAGTTGTC |
| 33 | GACAACTCACACCGCCAAACACAGAGACCGGGTTGGCGGCG |
| 34 | CTGTGAGGATGTTCGCGTAATCCTGCGGTTAGTACTGCAAAAAGTG C |
| 35 | GCACTTTTTGCAGTACTAACCGCAGGATTACGCGAACATCCTCACA G |
| 36 | CTTCGCTCTTGATCTTCGGATAGTCACAGAGGGATATCGGCTAG |
| 37 | CTAGCCGATATCCCTCTGTGACTATCCGAAGATCAAGAGCGAAG |
| 38 | GACGAGTCAGACAGGAGGCAGTCTTGGAACGGTGAAAAGCCTG C |
| 39 | GCAGGCTTTTTCACCGTTCCAAGACTGCCTCCTGTCTGACTCGTC |
| 40 | CGCTCTTGATCTTCGGATAGTGGTGGTATTGTGACTGGGGA |
| 41 | TCCCCAGTCACAATACCACCACTATCCGAAGATCAAGAGCG |
| 42 | CATATGGAGTGTTATTTGAAGGGGTCTTGGAACGGTGAAAAAGCCT GC |
| 43 | GCAGGCTTTTTCACCGTTCCAAGACCCCTTCAAATAACACTCCATAT G |
| 44 | CCGATACAGTGTCCAAGTACG |
| 45 | GAGTGTCTGAAGACAAGGCTTC |
| 46 | GACGACAATGCTGAGCTCCG |

### Example 2-3. Preparation of Saccharomyces cerevisiae-derived BTS1-inserted strain

To insert the *Saccharomyces cerevisiae*-derived BTS1 gene (hereinbelow, referred to as Sc.BTS1) into the chromosome of *Yarrowia lipolytica,* a polynucleotide of SEQ ID NO: 9 of BTS1 was obtained, based on a nucleotide sequence (YPL069C) registered in the Kyoto Encyclopedia of Genes and Genomes (KEGG). The gene was synthesized using the polynucleotide of BTS1 by Macrogen in the form of TEFINtp-Sc.BTS1-TDH3t (SEQ ID NO: 10). A cassette to be inserted into the LIG4(YALI0D21384g) gene site was designed using the URA3 gene (SEQ ID NO: 5) of Y. *lipolytica* as a selection marker.

PCR was performed for left homologous region, TEFINt promoter, Sc.BTS1 ORF, TDH3 terminator, URA3, repeat region, and right homologous region fragments using the synthesized Sc.BTS1 DNA and genomic DNA of KCCM12972P as templates, and primers of SEQ ID NO: 31 and SEQ ID NO: 32, SEQ ID NO: 33 and SEQ ID NO: 47, SEQ ID NO: 48 and SEQ ID NO: 49, SEQ ID NO: 50 and SEQ ID NO: 38, SEQ ID NO: 39 and SEQ ID NO: 40, SEQ ID NO: 41 and SEQ ID NO: 42, and SEQ ID NO: 43 and SEQ ID NO: 44, as shown in Table 5, respectively. PCR was performed by 35 cycles consisting of denaturation at 95°C for 1 min; annealing at 55°C for 1 min; and polymerization reaction at 72°C for 2 min. The resulting DNA fragments were prepared as a single cassette through overlap extension PCR.

The cassette thus prepared was introduced into the CC08-1023 strain by a heat shock method, and then colonies were obtained, which were formed on a solid medium (YLMM1) without uracil. Colonies in which cassette insertion into the genome was confirmed using primers of SEQ ID NO: 45 and SEQ ID NO: 46 were plated on a 5-FOA solid medium and cultured at 30°C for 3 days, and colonies grown on the 5-FOA solid medium were obtained to remove the URA3 marker.

**[Table 5]**

| SEQ ID NO. | Sequence (5'-3') |
|---|---|
| 47 | CAGCTCATCTATCTTGGCCTCCTGCGGTTAGTACTGCAAAAAGTGC |
| 48 | GCACTTTTTGCAGTACTAACCGCAGGAGGCCAAGATAGATGAGCTG |
| 49 | CTTCGCTCTTGATCTTCGGATAGTCACAATTCGGATAAGTGGTCTATTATATATAA C |
| 50 | GTTATATATAATAGACCACTTATCCGAATTGTGACTATCCGAAGATCAAGAGCGA AG |

### Example 2-4. Preparation of Yarrowia lipolytica-derived GGS1-inserted strain

To insert the *Yarrowia lipolytica-*derived GGS1 gene (hereinbelow, referred to as YI.GGS1) into the chromosome of *Yarrowia lipolytica,* a polynucleotide of SEQ ID NO: 11 of GGS1 was obtained, based on a nucleotide sequence (YALI0D17050g) registered in the Kyoto Encyclopedia of Genes and Genomes (KEGG). The gene was synthesized using the polynucleotide of YI.GGS1 in the form of TEFINtp-YI.GGS1-TDH3t (SEQ ID NO: 12). A cassette to be inserted into the LIG4(YALI0D21384g) gene site was designed using the URA3 gene (SEQ ID NO: 5) of Y. *lipolytica* as a selection marker.

PCR was performed for left homologous region, TEFINt promoter, YI.GGS1 ORF, TDH3 terminator, URA3, repeat region, and right homologous region fragments using the synthesized YI.GGS1 gene and genomic DNA of KCCM12972P as templates, and primers of SEQ ID NO: 31 and SEQ ID NO: 32, SEQ ID NO: 33 and SEQ ID NO: 51, SEQ ID NO: 52 and SEQ ID NO: 53, SEQ ID NO: 54 and SEQ ID NO: 38, SEQ ID NO: 39 and SEQ ID NO: 40, SEQ ID NO: 41 and SEQ ID NO: 42, and SEQ ID NO: 43 and SEQ ID NO: 44, as shown in Table 6, respectively. PCR was performed by 35 cycles consisting of denaturation at 95°C for 1 min; annealing at 55°C for 1 min; and polymerization reaction at 72°C for 2 min. The resulting DNA fragments were prepared as a single cassette through overlap extension PCR.

The cassette thus prepared was introduced into the CC08-1023 strain by a heat shock method, and then colonies were obtained, which were formed on a solid medium (YLMM1) without uracil. Colonies in which cassette insertion into the genome was confirmed using primers of SEQ ID NO: 45 and SEQ ID NO: 46 were plated on a 5-FOA solid medium and cultured at 30°C for 3 days, and colonies grown on the 5-FOA solid medium were obtained to remove the URA3 marker.

**[Table 6]**

| SEQ ID NO. | Sequence (5'-3') |
|---|---|
| 51 | CTTGAAATCCGCGCTGTTATAATCCTGCGGTTAGTACTGCAAAAAG TGC |
| 52 | GCACTTTTTGCAGTACTAACCGCAGGATTATAACAGCGCGGATTTC AAG |
| 53 | CTTCGCTCTTGATCTTCGGATAGTCACTGCGCATCCTCAAAGTAC |
| 54 | GTACTTTGAGGATGCGCAGTGACTATCCGAAGATCAAGAGCGAAG |

### Example 3. Comparative Evaluation of Beta-carotene Production Ability, based on GGPP Synthase-Introduced Strain

A flask test was performed on a total of 5 species, comprising the strains obtained in Examples 2-1 to 2-4 and the parent strain CC08-1023 obtained in Example 1. The strains were each inoculated at an initial OD of 2 in a 250 ml corner-baffle flask containing 20 ml of Yeast extract-Peptone-Dextrose (YPD) medium and cultured at 30°C for 48 hours with agitation at 200 rpm. After completion of the culture, 1 ml of the culture broth was centrifuged and the supernatant was removed. The composition of the YPD medium is as follows.

### <YPD liquid media >

4% glucose, 1% yeast extract, and 2% peptone dissolved in 0.1 M phosphate buffer (sodium phosphate buffer) (pH 7.0).

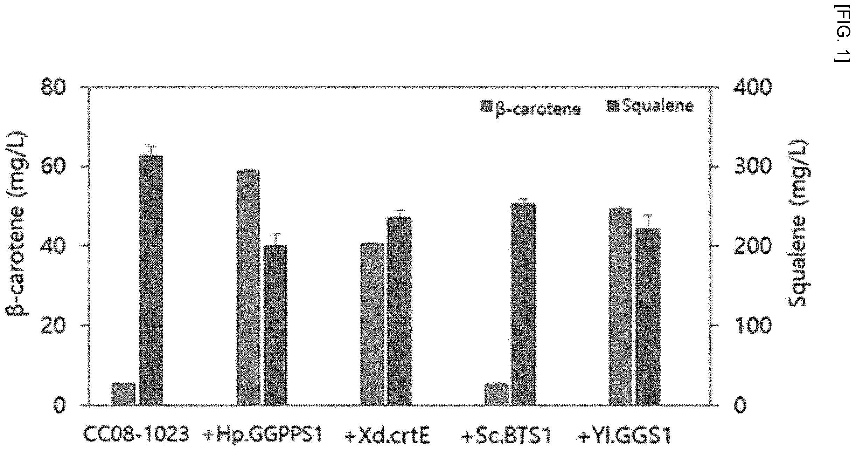

Next, 0.5 ml of dimethyl sulfoxide (DMSO, Sigma, CAS number 67-68-5) was added, and the cells were disrupted by agitation (2,000 rpm) for 10 minutes at 55°C. Additionally, 0.5 ml of acetone (Sigma, CAS number 67-64-1) was added and agitated (2,000 rpm) at 45°C for 15 minutes to extract beta-carotene and squalene, and concentrations thereof were analyzed using HPLC equipment. The results of measuring the analyzed beta-carotene and squalene concentrations are shown in FIG. 1.

As a result, as shown in FIG. 1, the beta-carotene concentrations in CC08-1023 (parent strain), Hp.GGPPS1-introduced strain, crtEM1-introduced strain, Sc.BTS1-introduced strain, and YI.GGS1-introduced strain were 5.49 mg/L, 58.73 mg/L, 40.58 mg/L, 5.21 mg/L, and 49.22 mg/L, respectively. In particular, when Hp.GGPPS1 was introduced, beta-carotene was increased by 53.24 mg/L, as compared to the parent strain, indicating the most excellent effect of increasing beta-carotene carotene.

Additionally, the squalene concentration was 313.24 mg/L, 200.31 mg/L, 235.27 mg/L, 253.28 mg/L, and 221.22 mg/L, respectively. Similarly, when Hp.GGPPS1 was introduced, squalene was reduced by 112.93 mg/L, as compared to the CC08-1023 strain, indicating the most excellent effect of reducing squalene.

Based on these results, it was confirmed that Hp.GGPPS1 is the most effective as GGPP synthase in microorganisms of the genus *Yarrowia.* Surprisingly, when the geranylgeranyl pyrophosphate synthase derived from the closely related *Saccharomyces cerevisiae, Yarrowia lipolytica,* and *Xanthophyllomyces dendrorhous* was introduced, the effect was insignificant, whereas when the geranylgeranyl pyrophosphate synthase derived from the relatively unrelated *Haematococcus pluvialis* was introduced, the effect was remarkable.

### Example 4. Preparation of Beta-carotene 15,15'oxygenase(BCO) gene-introduced Strain

To insert the *Uncultured marine bacterium* 66A03-derived beta-carotene 15,15'oxygenase (hereinbelow, referred to as Mb.BCO) gene into the chromosome of *Yarrowia lipolytica,* a polynucleotide sequence (SEQ ID NO: 13) was obtained by codon optimization of Mb.BCO to be suitable for Y. *lipolytica* through http://atgme.org, based on an amino acid sequence (Q4PNI0) registered in UniProt Knowledgebase (UniProtKB). The gene was synthesized using the polynucleotide of Mb.BCO in the form of TEFINtp-codon optimized Mb.BCO-CYC1t (SEQ ID NO: 14). A cassette to be inserted into the KU70(YALI0C08701g) gene site was designed using the URA3 gene (SEQ ID NO: 5) of Y. *lipolytica* as a selection marker.

PCR was performed for left homologous region, TEFINt promoter, Mb.BCO ORF, CYC1 terminator, URA3, repeat region, and right homologous region using the synthesized Mb.BCO and genomic DNA of KCCM12972P as templates, and primers of SEQ ID NO: 55 and SEQ ID NO: 56, SEQ ID NO: 57 and SEQ ID NO: 58, SEQ ID NO: 59 and SEQ ID NO: 60, SEQ ID NO: 61 and SEQ ID NO: 62, SEQ ID NO: 63 and SEQ ID NO: 64, SEQ ID NO: 65 and SEQ ID NO: 66, and SEQ ID NO: 67 and SEQ ID NO: 68, as shown in Table 7, respectively. PCR was performed by 35 cycles consisting of denaturation at 95°C for 1 min; annealing at 55°C for 1 min; and polymerization reaction at 72°C for 2 min. The resulting DNA fragments were prepared as a single cassette through overlap extension PCR.

The cassette thus prepared was introduced into each of the strains prepared in Examples 2-1 to 2-4 by a heat shock method, and then colonies were obtained, which were formed on a solid medium (YLMM1) without uracil. Colonies in which cassette insertion into the genome was confirmed using primers of SEQ ID NO: 69 and SEQ ID NO: 70 were plated on a 5-FOA solid medium and cultured at 30°C for 3 days, and colonies grown on the 5-FOA solid medium were obtained to remove the URA3 marker.

**[Table 7]**

| SEQ ID NO. | Sequence (5'-3') |
|---|---|
| 55 | GGCGTTTCAGGTGGTTGCGTGAGTG |
| 56 | GACACAAATGCGCCGCCAACCCGGTCTCTGCGGCGGTTCGTGGTTC GTGTTTC |
| 57 | GAAACACGAACCACGAACCGCCGCAGAGACCGGGTTGGCGGCGCAT |
| | TTGTGTC |
| 58 | CAGTCGATCAGCATCAGGCCCTGCGGTTAGTACTGCAAAA |
| 59 | TTTTGCAGTACTAACCGCAGGGCCTGATGCTGATCGACTG |
| 60 | AACTAATTACATGActcgaGCTAGTTCTTGATCTTGATTC |
| 61 | GAATCAAGATCAAGAACTAGCtcgagTCATGTAATTAGTT |
| 62 | gacgagtcagacaggaggcaGCAAATTAAAGCCTTCGAGCGTCCC |
| 63 | GGGACGCTCGAAGGCTTTAATTTGCtgcctcctgtctgactcgtc |
| 64 | AACTAATTACATGActcgaGtggtggtattgtgactgggg |
| 65 | ccccagtcacaataccaccaCtcgagTCATGTAATTAGTT |
| 66 | GCAGCAGTCATACATGTTCTGAGGCAAATTAAAGCCTTCGAGCGTCCC |
| 67 | GGGACGCTCGAAGGCTTTAATTTGCCTCAGAACATGTATGACTGCTGC |
| 68 | CTACTTTGTGCAGATTGAGGCCAAG |
| 69 | GTCGTCTGTCTTCTCTTCAG |
| 70 | CCACCAAGATGGGCAAGAAG |

### Example 5. Comparative Evaluation of Retinol Production Ability of Beta-carotene 15,15'oxygenase(BCO) gene-introduced Strain

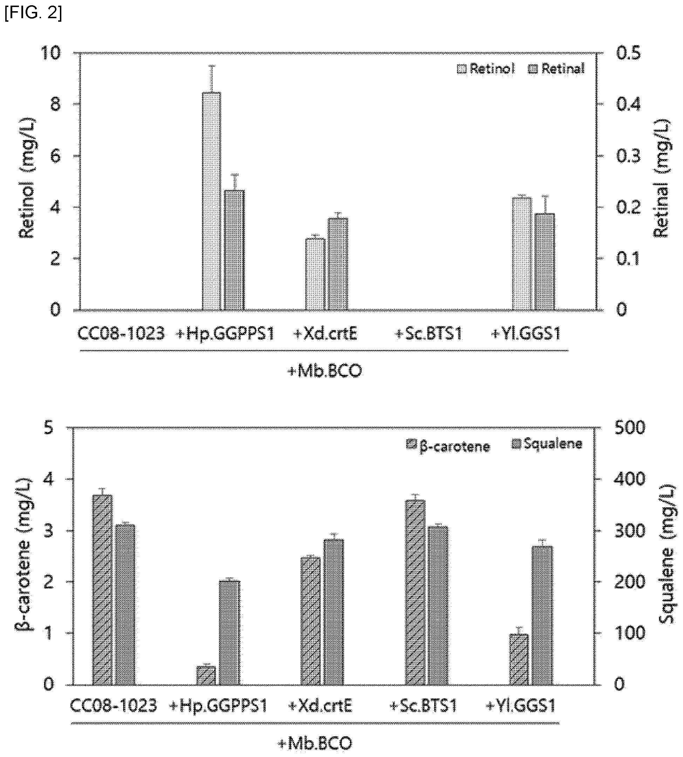

A flask test was performed on a total of 5 species, comprising the strain obtained in Example 4 and the parent strain CC08-1023 obtained in Example 1. The strains were each inoculated at an initial OD of 2 in a 250 ml corner-baffle flask containing 20 ml of Yeast extract-Peptone-Dextrose (YPD) medium and 0.05% butylated hydroxytoluene, and cultured at 30°C for 48 hours with agitation at 200 rpm. After completion of the culture, 1 ml of the culture medium was centrifuged and the supernatant was removed. Next, 0.5 ml of dimethyl sulfoxide (DMSO, Sigma) was added, and the cells were disrupted by agitation (2,000 rpm) for 10 minutes at 55°C. Additionally, 0.5 ml of acetone (Sigma) was added and agitated (2,000 rpm) at 45°C for 15 minutes to extract retinol, retinal, beta-carotene, and squalene, and concentrations thereof were analyzed using HPLC equipment. The results of measuring the analyzed retinol, retinal, beta-carotene, and squalene concentrations are shown in FIG. 2.

As a result, as shown in FIG. 2, retinol was not measured in the strain prepared by introducing Mb.BCO into the CC08-1023 strain. In contrast, the retinol concentrations in four types of strains into which Mb.BCO was introduced after introducing each of Hp.GGPPS1, crtEM1, Sc.BTS1, and YI,GGS1, based on CC08-1023, were 8.44 mg/L, 2.78 mg/L, 0 mg/L, and 4.35 mg/L, respectively.

The beta-carotene concentrations in the five types of strains were 3.68 mg/L, 0.35 mg/L, 2.47 mg/L, 3.58 mg/L, and 0.98 mg/L, respectively, indicating that beta-carotene was converted to retinol, resulting in the low beta-carotene concentration. In addition, the squalene concentrations in the five types of strains were 309.88 mg/L, 202.18 mg/L, 282.19 mg/L, 306.34 mg/L, and 269.18 mg/L, respectively.

These results confirmed that the enhancement of GGPP biosynthesis had a positive effect on increasing the retinol productivity.

The above results also verified that Hp.GGPPS1 has the excellent effects on beta-carotene production, squalene reduction, and retinol production.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

Each sequence according to SEQ ID NO. of the present disclosure is shown in Table 8 below.

**[Table 8]**

| SEQ ID NO. | Name | Sequence | Type |
|---|---|---|---|
| 1 | Codon optimized Hp.GGPPS1 ORF | atgatccgag ccatgcacaa ccgagccccc accccccgaa cccgagtgtc tcacccccga 60 tctcaccgag ccctggccca cgtgtctgcc gtggccaccg ccggccaggt ggccgaggtg 120 cactctgccc ccgccttcga cttcgagatg tacatgcgag accgagccga gatggtgaac 180 aaggccctgg acgccgccct gccctctcga taccccgagg tgctggtgga ctctatgcga 240 tactctgtgc tggccggcgg caagcgagtg cgacccgccc tgaccctggc cgcctgtgac 300 ctggtgggcg gcgacatggc caccgccctg cccaccgcct gtgccatgga gatgatccac 360 accatgtctc tgatccacga cgacctgccc gccatggaca acgacgactt ccgacgaggc 420 cgacccacca accacaaggt gtacggcgag gacatcgcca tcctggccgg cgacgccctg 480 ctgtctttcg ccttcgagca catcgcccga gacaccaagg | DNA |
| | | gcgtgcccgc cgacgccgtg 540 ctgaaggtga tcatggagct gggccgagcc gtgggcgccc agggcctgtc tgccggccag 600 gccgtggaca tcaagtctga gggccaggag gtgggcctgg aggtgctgga gtacatccac 660 caccacaaga ccgccgccct gctggaggcc gccgtggtgt gtggcgccct ggtgggcggc 720 gccgacaccg ccaccgtgga gaagctgcga aagtacgccc tgaacatcgg cctggccttc 780 caggtgatcg acgacatcct ggacgtgacc cagaccaccg agaccctggg caagaccgcc 840 gccaaggacc tggccgtgaa caagaccacc taccccaagc tgctgggcct ggaggcctct 900 cgaaaggtgg ccgacgacct gatccgagag gccatcgccc agctggacga gttcgagccc 960 gcccgaaagg cccccatggt ggccctggcc cacctgatcg gctaccgaaa gaactag | |
| 2 | TEFINtp | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca g | DNA |
| 3 | CYC1t | ctcgagtcat gtaattagtt atgtcacgct tacattcacg ccctcccccc acatccgctc 60 taaccgaaaa ggaaggagtt agacaacctg aagtctaggt ccctatttatt tttttatag 120 ttatgttagt attaagaacg ttatttatat ttcaaatttt tctttttttt ctgtacagac 180 gcgtgtacgc atgtaacatt atactgaaaa ccttgcttga gaaggttttg ggacgctcga 240 aggctttaat ttgc | DNA |
| 4 | TEFINtp-codon optimized Hp.GGPPS1-CYC1t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg | DNA |
| | | tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca gatccgagcc 540 atgcacaacc gagcccccac cccccgaacc cgagtgtctc acccccgatc tcaccgagcc 600 ctggcccacg tgtctgccgt ggccaccgcc ggccaggtgg ccgaggtgca ctctgccccc 660 gccttcgact tcgagatgta catgcgagac cgagccgaga tggtgaacaa ggccctggac 720 gccgccctgc cctctcgata ccccgaggtg ctggtggact ctatgcgata ctctgtgctg 780 gccggcggca agcgagtgcg acccgccctg accctggccg cctgtgacct ggtgggcggc 840 | |
| | | gacatggcca ccgccctgcc caccgcctgt gccatggaga tgatccacac catgtctctg 900 atccacgacg acctgcccgc catggacaac gacgacttcc gacgaggccg acccaccaac 960 cacaaggtgt acggcgagga catcgccatc ctggccggcg acgccctgct gtctttcgcc 1020 ttcgagcaca tcgcccgaga caccaagggc gtgcccgccg acgccgtgct gaaggtgatc 1080 atggagctgg gccgagccgt gggcgcccag ggcctgtctg ccggccaggc cgtggacatc 1140 aagtctgagg gccaggaggt gggcctggag gtgctggagt acatccacca ccacaagacc 1200 gccgccctgc tggaggccgc cgtggtgtgt ggcgccctgg tgggcggcgc cgacaccgcc 1260 accgtggaga agctgcgaaa gtacgccctg aacatcggcc tggccttcca ggtgatcgac 1320 gacatcctgg acgtgaccca gaccaccgag accctgggca agaccgccgc caaggacctg 1380 gccgtgaaca agaccaccta ccccaagctg ctgggcctgg aggcctctcg aaaggtggcc 1440 gacgacctga tccgagaggc catcgcccag ctggacgagt tcgagcccgc ccgaaaggcc 1500 cccatggtgg ccctggccca cctgatcggc taccgaaaga actagctcga gtcatgtaat 1560 tagttatgtc acgcttacat tcacgccctc cccccacatc cgctctaacc gaaaaggaag 1620 gagttagaca acctgaagtc taggtcccta tttatttttt tatagttatg ttagtattaa 1680 gaacgttatt tatatttcaa atttttcttt tttttctgta cagacgcgtg tacgcatgta 1740 acattatact gaaaaccttg cttgagaagg ttttgggacg ctcgaaggct ttaatttgc | |
| 5 | URA3 | tgcctcctgt ctgactcgtc attgccgcct ttggagtacg actccaacta tgagtgtgct 60 tggatcactt tgacgataca ttcttcgttg gaggctgtgg gtctgacagc tgcgttttcg 120 gcgcggttgg ccgacaacaa tatcagctgc aacgtcattg | DNA |
| | | ctggctttca tcatgatcac 180 atttttgtcg gcaaaggcga cgcccagaga gccattgacg ttctttctaa tttggaccga 240 tagccgtata gtccagtcta tctataagtt caactaactc gtaactatta ccataacata 300 tacttcactg ccccagataa ggttccgata aaaagttctg cagactaaat ttatttcagt 360 ctcctcttca ccaccaaaat gccctcctac gaagctcgag ctaacgtcca caagtccgcc 420 tttgccgctc gagtgctcaa gctcgtggca gccaagaaaa ccaacctgtg tgcttctctg 480 gatgttacca ccaccaagga gctcattgag cttgccgata aggtcggacc ttatgtgtgc 540 atgatcaaga cccatatcga catcattgac gacttcacct acgccggcac tgtgctcccc 600 ctcaaggaac ttgctcttaa gcacggtttc ttcctgttcg aggacagaaa gttcgcagat 660 attggcaaca ctgtcaagca ccagtacaag aacggtgtct accgaatcgc cgagtggtcc 720 gatatcacca acgcccacgg tgtacccgga accggaatca ttgctggcct gcgagctggt 780 gccgaggaaa ctgtctctga acagaagaag gaggacgtct ctgactacga gaactcccag 840 tacaaggagt tcctggtccc ctctcccaac gagaagctgg ccagaggtct gctcatgctg 900 gccgagctgt cttgcaaggg ctctctggcc actggcgagt actccaagca gaccattgag 960 cttgcccgat ccgaccccga gtttgtggtt ggcttcattg cccagaaccg acctaagggc 1020 gactctgagg actggcttat tctgaccccc ggggtgggtc ttgacgacaa gggagacgct 1080 ctcggacagc agtaccgaac tgttgaggat gtcatgtcta ccggaacgga tatcataatt 1140 gtcggccgag gtctgtacgg ccagaaccga gatcctattg aggaggccaa gcgataccag 1200 aaggctggct gggaggctta ccagaagatt aactgttaga ggttagacta tggatatgtc 1260 atttaactgt gtatatagag agcgtgcaag tatggagcgc ttgttcagct tgtatgatgg 1320 tcagacgacc tgtctgatcg agtatgtatg atactgcaca acctgtgtat ccgcatgatc 1380 tgtccaatgg ggcatgttgt tgtgtttctc gatacggaga tgctgggtac aagtagctaa 1440 tacgattgaa ctacttatac ttatatgagg cttgaagaaa gctgacttgt gtatgactta 1500 ttctcaacta catccccagt cacaatacca cca | |
| 6 | Codon optimized crtEM1 ORF | atggattacg cgaacatcct cacagcaatt ccactcgagt ttactcctca ggatgatatc 60 gtgctccttg aaccgtatca ctacctagga aagaaccctg gaaaagaaat tcgatcacaa 120 ctcatcgagg ctttcaacta ttggttggat gtcaagaagg aggatctcga ggtcatccag 180 aacgttgttg gcatgctaca taccgctagc ttattaatgg acgatgtgga ggattcatcg 240 gtcctcaggc gtgggtcgcc tgtagcccat ctaatttacg ggattccgca | DNA |
| | | gacaataaac 300 actgcaaact acgtctactt tctggcttat caagagatct tcaagcttcg cccaacaccg 360 atacccatgc ctgtaattcc tccttcatct gcttcgcttc aatcaaccgt ctcctctgca 420 tcctcctcct cctcggcctc gtctgaaaac gggggcacgt catctcctaa ttcgcagatt 480 ccgttctcga aagatacgta tcttgataaa gtgatcacag acgagatgct ttccctccat 540 agagggcaag gcctggagct attctggaga gatagtctga cgtgtcctag cgaagaggaa 600 tatgtgaaaa tggttcttgg aaagacggga ggtttgttcc gtatagcggt cagattgatg 660 atggcaaagt cagaatgtga catagacttt gtccagcttg tcaacttgat ctcaatatac 720 ttccagatca gggatgacta tatgaacctt cagtcttctg agtatgccca tattaagaat 780 tttgcagagg acctcacaga aggaaaattc agttttccca ctatccactc gattcgtgcc 840 aacccctcat cgagactcgt catcaatacg ttgcagaaga aatcgacctc tcctgagatc 900 cttcaccact gtgtaaacta catgcgcaca gaaacccact cattcgaata tactcaggaa 960 gtcctcaaca ccttgtcagg tgcactcgag agagaactag gaaggcttca aggagagttc 1020 gcagaagcta actcaaagat tgatcttgga gacgtagagt cggaaggaag aacggggaag 1080 aacgtcaaat tggaagcgat cctgaaaaag ctagccgata tccctctgtg a | |
| 7 | TDH3t | ctatccgaag atcaagagcg aagcaagttg taagtccagg acatgtttcc cgcccacgcg 60 agtgatttat aacacctctct tttttgaca cccgctcgcc ttgaaattca tgtcacataa 120 attatagtca acgacgtttg aataacttgt cttgtagttc gatgatgatc atatgattac 180 attaatagta attactgtat ttgatatata tactaattac aatagtacat attagaacat 240 acaatagtta gtgccgtgaa gtggcttaaa ataccgcgag tcgattacgt aatattatat 300 ataatgtcaa agtggggtcc cagagccgaa gaaggtgctt ttcttgaaga tcccagtgta 360 ttggacaagt atatctgtct ctatgattgt ttttccaggt gaaaatgttg aacaaagtgt 420 ctactggagt ttgtaagcgc tggtgcgact ggggccactt ttaaaacccg ccttagcagg 480 ctttttcacc gttccaagac | DNA |
| 8 | TEFINtp-codon optimized crtEM1-TDH3t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca ggattacgcg 540 aacatcctca cagcaattcc actcgagttt actcctcagg atgatatcgt gctccttgaa 600 ccgtatcact acctaggaaa gaaccctgga aaagaaattc gatcacaact catcgaggct 660 ttcaactatt ggttggatgt caagaaggag gatctcgagg tcatccagaa cgttgttggc 720 atgctacata ccgctagctt attaatggac gatgtggagg attcatcggt cctcaggcgt 780 gggtcgcctg tagcccatct aatttacggg attccgcaga caataaacac tgcaaactac 840 gtctactttc tggcttatca agagatcttc aagcttcgcc caacaccgat acccatgcct 900 gtaattcctc cttcatctgc ttcgcttcaa tcaaccgtct cctctgcatc ctcctcctcc 960 tcggcctcgt ctgaaaacgg gggcacgtca tctcctaatt cgcagattcc gttctcgaaa 1020 gatacgtatc ttgataaagt gatcacagac gagatgcttt ccctccatag agggcaaggc 1080 ctggagctat tctggagaga tagtctgacg tgtcctagcg aagaggaata tgtgaaaatg 1140 gttcttggaa agacgggagg tttgttccgt atagcggtca gattgatgat ggcaaagtca 1200 gaatgtgaca tagactttgt ccagcttgtc aacttgatct caatatactt ccagatcagg 1260 gatgactata tgaaccttca gtcttctgag tatgcccata ttaagaattt tgcagaggac 1320 ctcacagaag gaaaattcag ttttcccact atccactcga ttcgtgccaa cccctcatcg 1380 agactcgtca tcaatacgtt gcagaagaaa tcgacctctc ctgagatcct tcaccactgt 1440 gtaaactaca tgcgcacaga aacccactca ttcgaatata ctcaggaagt cctcaacacc 1500 ttgtcaggtg cactcgagag agaactagga aggcttcaag gagagttcgc agaagctaac 1560 tcaaagattg atcttggaga cgtagagtcg gaaggaagaa cggggaagaa cgtcaaattg 1620 | DNA |
| | | gaagcgatcc tgaaaaagct agccgatatc cctctgtgac tatccgaaga tcaagagcga 1680 agcaagttgt aagtccagga catgtttccc gcccacgcga gtgatttata acacctctct 1740 tttttgacac ccgctcgcct tgaaattcat gtcacataaa ttatagtcaa cgacgtttga 1800 ataacttgtc ttgtagttcg atgatgatca tatgattaca ttaatagtaa ttactgtatt 1860 tgatatatat actaattaca atagtacata ttagaacata caatagttag | |
| | | tgccgtgaag 1920 tggcttaaaa taccgcgagt cgattacgta atattatata taatgtcaaa gtggggtccc 1980 agagccgaag aaggtgcttt tcttgaagat cccagtgtat tggacaagta tatctgtctc 2040 tatgattgtt tttccaggtg aaaatgttga acaaagtgtc tactggagtt tgtaagcgct 2100 ggtgcgactg gggccacttt taaaacccgc cttagcaggc tttttcaccg ttccaagac | |
| 9 | Sc.BTS1 ORF | atggaggcca agatagatga gctgatcaat aatgatcctg tttggtccag ccaaaatgaa 60 agcttgattt caaaacctta taatcacatc cttttgaaac ctggcaagaa ctttagacta 120 aatttaatag ttcaaattaa cagagttatg aatttgccca aagaccagct ggccatagtt 180 tcgcaaattg ttgagctctt gcataattcc agccttttaa tcgacgatat agaagataat 240 gctcccttga gaaggggaca gaccacttct cacttaatct tcggtgtacc ctccactata 300 aacaccgcaa attatatgta tttcagagcc atgcaacttg tatcgcagct aaccacaaaa 360 gagcctttgt atcataattt gattacgatt ttcaacgaag aattgatcaa tctacatagg 420 ggacaaggct tggatatata ctggagagac tttctgcctg aaatcatacc tactcaggag 480 atgtatttga atatggttat gaataaaaca ggcggccttt tcagattaac gttgagactc 540 atggaagcgc tgtctccttc ctcacaccac ggccattcgt tggttccttt cataaatctt 600 ctgggtatta tttatcagat tagagatgat tacttgaatt tgaaagattt ccaaatgtcc 660 agcgaaaaag gctttgctga ggacattaca gaggggaagt tatcttttcc catcgtccac 720 gcccttaact tcactaaaac gaaaggtcaa actgagcaac acaatgaaat tctaagaatt 780 ctcctgttga ggacaagtga taaagatata aaactaaagc tgattcaaat actggaattc 840 gacaccaatt cattggccta caccaaaaat tttattaatc aattagtgaa tatgataaaa 900 aatgataatg aaaataagta tttacctgat ttggcttcgc attccgacac cgccaccaat 960 ttacatgacg aattgttata tataatagac cacttatccg aattgtga | DNA |
| 10 | TEFINtp-Sc.BTS1-TDH3t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg | DNA |
| | | tgagtttcag 420 aggcagcagc aattgccacg ggctttgagc acacggccgg gtgtggtccc attcccatcg 480 acacaagacg ccacgtcatc cgaccagcac tttttgcagt actaaccgca ggaggccaag 540 atagatgagc tgatcaataa tgatcctgtt tggtccagcc aaaatgaaag cttgatttca 600 aaaccttata atcacatcct tttgaaacct ggcaagaact ttagactaaa tttaatagtt 660 caaattaaca gagttatgaa tttgcccaaa gaccagctgg ccatagtttc gcaaattgtt 720 gagctcttgc ataattccag ccttttaatc gacgatatag aagataatgc tcccttgaga 780 aggggacaga ccacttctca cttaatcttc ggtgtaccct ccactataaa caccgcaaat 840 tatatgtatt tcagagccat gcaacttgta tcgcagctaa ccacaaaaga gcctttgtat 900 cataatttga ttacgatttt caacgaagaa ttgatcaatc tacatagggg acaaggcttg 960 gatatatact ggagagactt tctgcctgaa atcataccta ctcaggagat gtatttgaat 1020 atggttatga ataaaacagg cggccttttc agattaacgt tgagactcat ggaagcgctg 1080 tctccttcct cacaccacgg ccattcgttg gttcctttca taaatcttct gggtattatt 1140 tatcagatta gagatgatta cttgaatttg aaagatttcc aaatgtccag cgaaaaaggc 1200 tttgctgagg acattacaga ggggaagtta tcttttccca tcgtccacgc ccttaacttc 1260 actaaaacga aaggtcaaac tgagcaacac aatgaaattc taagaattct cctgttgagg 1320 acaagtgata aagatataaa actaaagctg attcaaatac tggaattcga caccaattca 1380 ttggcctaca ccaaaaattt tattaatcaa ttagtgaata tgataaaaaa tgataatgaa 1440 aataagtatt tacctgattt ggcttcgcat tccgacaccg ccaccaattt acatgacgaa 1500 ttgttatata taatagacca cttatccgaa ttgtgactat ccgaagatca agagcgaagc 1560 aagttgtaag tccaggacat gtttcccgcc cacgcgagtg atttataaca cctctctttt 1620 | |
| | | ttgacacccg ctcgccttga aattcatgtc acataaatta tagtcaacga cgtttgaata 1680 acttgtcttg tagttcgatg atgatcatat gattacatta atagtaatta ctgtatttga 1740 tatatatact aattacaata gtacatatta gaacatacaa tagttagtgc cgtgaagtgg 1800 cttaaaatac cgcgagtcga ttacgtaata ttatatataa tgtcaaagtg gggtcccaga 1860 gccgaagaag gtgcttttct tgaagatccc agtgtattgg acaagtatat ctgtctctat 1920 gattgttttt ccaggtgaaa atgttgaaca aagtgtctac tggagtttgt aagcgctggt 1980 gcgactgggg ccacttttaa aacccgcctt agcaggcttt ttcaccgttc caagac | |
| 11 | YI.GGS1 ORF | atggattata acagcgcgga tttcaaggag atatggggca aggccgccga caccgcgctg 60 ctgggaccgt acaactacct cgccaacaac cggggccaca acatcagaga acacttgatc 120 gcagcgttcg gagcggttat caaggtggac aagagcgatc tcgagaccat ttcgcacatc 180 accaagattt tgcataactc gtcgctgctt gttgatgacg tggaagacaa ctcgatgctc 240 cgacgaggcc tgccggcagc ccattgtctg tttggagtcc cccaaaccat caactccgcc 300 aactacatgt actttgtggc tctgcaggag gtgctcaagc tcaagtctta tgatgccgtc 360 tccattttca ccgaggaaat gatcaacttg catagaggtc agggtatgga tctctactgg 420 agagaaacac tcacttgccc ctcggaagac gagtatctgg agatggtggt gcacaagacc 480 ggtggactgt ttcggctggc tctgagactt atgctgtcgg tggcatcgaa acaggaggac 540 catgaaaaga tcaactttga tctcacacac cttaccgaca cactgggagt catttaccag 600 attctggatg attacctcaa cctgcagtcc acggaattga ccgagaacaa gggattctgc 660 gaagatatca gcgaaggaaa gttttcgttt ccgctgattc acagcatacg caccaacccg 720 gataaccacg agattctcaa cattctcaaa cagcgaacaa gcgacgcttc actcaaaaag 780 tacgccgtgg actacatgag aacagaaacc aagagtttcg actactgcct caagaggata 840 caggccatgt cactcaaggc aagttcgtac attgatgatc tagcagcagc tggccacgat 900 gtctccaagc tacgagccat tttgcattat tttgtgtcca cctctgactg tgaggagaga 960 aagtactttg aggatgcgca gtga | DNA |
| 12 | TEFINtp-YI.GGS1-TDH3t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca ggattataac 540 agcgcggatt tcaaggagat atggggcaag gccgccgaca ccgcgctgct gggaccgtac 600 aactacctcg ccaacaaccg gggccacaac atcagagaac acttgatcgc agcgttcgga 660 | DNA |
| | | gcggttatca aggtggacaa gagcgatctc gagaccattt cgcacatcac caagattttg 720 cataactcgt cgctgcttgt tgatgacgtg gaagacaact cgatgctccg acgaggcctg 780 ccggcagccc attgtctgtt tggagtcccc caaaccatca actccgccaa ctacatgtac 840 tttgtggctc tgcaggaggt gctcaagctc aagtcttatg atgccgtctc cattttcacc 900 gaggaaatga tcaacttgca tagaggtcag ggtatggatc tctactggag agaaacactc 960 acttgcccct cggaagacga gtatctggag atggtggtgc acaagaccgg tggactgttt 1020 cggctggctc tgagacttat gctgtcggtg gcatcgaaac aggaggacca tgaaaagatc 1080 aactttgatc tcacacacct taccgacaca ctgggagtca tttaccagat tctggatgat 1140 tacctcaacc tgcagtccac ggaattgacc gagaacaagg gattctgcga agatatcagc 1200 gaaggaaagt tttcgtttcc gctgattcac agcatacgca ccaacccgga taaccacgag 1260 attctcaaca ttctcaaaca gcgaacaagc gacgcttcac tcaaaaagta cgccgtggac 1320 tacatgagaa cagaaaccaa gagtttcgac tactgcctca agaggataca ggccatgtca 1380 ctcaaggcaa gttcgtacat tgatgatcta gcagcagctg gccacgatgt ctccaagcta 1440 cgagccattt tgcattattt tgtgtccacc tctgactgtg aggagagaaa gtactttgag 1500 | |
| | | gatgcgcagt gactatccga agatcaagag cgaagcaagt tgtaagtcca ggacatgttt 1560 cccgcccacg cgagtgattt ataacacctc tcttttttga cacccgctcg ccttgaaatt 1620 catgtcacat aaattatagt caacgacgtt tgaataactt gtcttgtagt tcgatgatga 1680 tcatatgatt acattaatag taattactgt atttgatata tatactaatt acaatagtac 1740 atattagaac atacaatagt tagtgccgtg aagtggctta aaataccgcg agtcgattac 1800 gtaatattat atataatgtc aaagtggggt cccagagccg aagaaggtgc ttttcttgaa 1860 gatcccagtg tattggacaa gtatatctgt ctctatgatt gtttttccag gtgaaaatgt 1920 tgaacaaagt gtctactgga gtttgtaagc gctggtgcga ctggggccac ttttaaaacc 1980 cgccttagca ggctttttca ccgttccaag ac | |
| 13 | Codon optimized Mb.BCO ORF | atgggcctga tgctgatcga ctggtgtgcc ctggccctgg tggtgttcat cggcctgccc 60 cacggcgccc tggacgccgc catctctttc tctatgatct cttctgccaa gcgaatcgcc 120 cgactggccg gcatcctgct gatctacctg ctgctggcca ccgccttctt cctgatctgg 180 taccagctgc ccgccttctc tctgctgatc ttcctgctga tctctatcat ccacttcggc 240 atggccgact tcaacgcctc tccctctaag ctgaagtggc cccacatcat cgcccacggc 300 | DNA |
| | | ggcgtggtga ccgtgtggct gcccctgatc cagaagaacg aggtgaccaa gctgttctct 360 atcctgacca acggccccac ccccatcctg tgggacatcc tgctgatctt cttcctgtgt 420 tggtctatcg gcgtgtgtct gcacacctac gagaccctgc gatctaagca ctacaacatc 480 gccttcgagc tgatcggcct gatcttcctg gcctggtacgc cccccccct ggtgaccttc 540 gccacctact tctgtttcat ccactctcga cgacacttct ctttcgtgtg gaagcagctg 600 cagcacatgt cttctaagaa gatgatgatc ggctctgcca tcatcctgtc ttgtacctct 660 tggctgatcg gcggcggcat ctacttcttc ctgaactcta agatgatcgc ctctgaggcc 720 gccctgcaga ccgtgttcat cggcctggcc gccctgaccg tgccccacat gatcctgatc 780 gacttcatct tccgacccca ctcttctcga atcaagatca agaactag | |
| 14 | TEFINtp-codon optimized Mb.BCO-CYC1t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca gggcctgatg 540 ctgatcgact ggtgtgccct ggccctggtg gtgttcatcg gcctgcccca cggcgccctg 600 gacgccgcca tctctttctc tatgatctct tctgccaagc gaatcgcccg actggccggc 660 atcctgctga tctacctgct gctggccacc gccttcttcc tgatctggta ccagctgccc 720 gccttctctc tgctgatctt cctgctgatc tctatcatcc acttcggcat ggccgacttc 780 aacgcctctc cctctaagct gaagtggccc cacatcatcg cccacggcgg cgtggtgacc 840 gtgtggctgc ccctgatcca gaagaacgag gtgaccaagc tgttctctat cctgaccaac 900 ggccccaccc ccatcctgtg ggacatcctg ctgatcttct tcctgtgttg gtctatcggc 960 gtgtgtctgc acacctacga gaccctgcga tctaagcact acaacatcgc cttcgagctg 1020 atcggcctga tcttcctggc ctggtacgcc ccccccctgg tgaccttcgc cacctacttc 1080 tgtttcatcc actctcgacg acacttctct ttcgtgtgga agcagctgca gcacatgtct 1140 | DNA |
| | | tctaagaaga tgatgatcgg ctctgccatc atcctgtctt gtacctcttg gctgatcggc 1200 ggcggcatct acttcttcct gaactctaag atgatcgcct ctgaggccgc cctgcagacc 1260 gtgttcatcg gcctggccgc cctgaccgtg ccccacatga tcctgatcga cttcatcttc 1320 cgaccccact cttctcgaat caagatcaag aactagctcg agtcatgtaa ttagttatgt 1380 cacgcttaca ttcacgccct ccccccacat ccgctctaac cgaaaaggaa ggagttagac 1440 aacctgaagt ctaggtccct atttattttt ttatagttat gttagtatta agaacgttat 1500 ttatatttca aatttttctt ttttttctgt acagacgcgt gtacgcatgt aacattatac 1560 tgaaaacctt gcttgagaag gttttgggac gctcgaaggc tttaatttgc | |
| 15 | primer | catcatttca aaagagggaa cagc | DNA |
| 16 | primer | cgccqccaac ccggtctctg tgtttggcgg tgtgagttgt c | DNA |
| 17 | primer | gacaactcac accgccaaac acagagaccg ggttggcggc g | DNA |
| 18 | primer | cggttgtgca tggctcggat ctgcggttag tactgcaaaa agtgc | DNA |
| 19 | primer | gcactttttg cagtactaac cgcagatccg agccatgcac aaccg | DNA |
| 20 | primer | aactaattac atgactcgag ctagttcttt cggtagccga | DNA |
| 21 | primer | tcggctaccg aaagaactag ctcgagtcat gtaattagtt | DNA |
| 22 | primer | gacgagtcag acaggaggca gcaaattaaa gccttcgagc | DNA |
| 23 | primer | gctcgaaggc tttaatttgc tgcctcctgt ctgactcgtc | DNA |
| 24 | primer | aactaattac atgactcgag tggtggtatt gtgactgggg | DNA |
| 25 | primer | ccccagtcac aataccacca ctcgagtcat gtaattagtt | DNA |
| 26 | primer | ccatatggag tgttatttga aggggcaaat taaagccttc gagc | DNA |
| 27 | primer | gctcgaaggc tttaatttgc cccttcaaat aacactccat atgg | DNA |
| 28 | primer | ccgatacagt gtccaagtac g | DNA |
| 29 | primer | gagtgtctga agacaaggct tc | DNA |
| 30 | primer | gacgacaatg ctgagctccg | DNA |
| 31 | primer | catcatttca aaagagggaa cagc | DNA |
| 32 | primer | cgccgccaac ccggtctctg tgtttggcgg tgtgagttgt c | DNA |
| 33 | primer | gacaactcac accgccaaac acagagaccg ggttggcggc g | DNA |
| 34 | primer | ctgtgaggat gttcgcgtaa tcctgcggtt agtactgcaa aaagtgc | DNA |
| 35 | primer | gcactttttg cagtactaac cgcaggatta cgcgaacatc ctcacag | DNA |
| 36 | primer | cttcgctctt gatettcgga tagtcacaga gggatatcgg ctag | DNA |
| 37 | primer | ctagccgata tccctctgtg actatccgaa gatcaagagc gaag | DNA |
| 38 | primer | qacqaqtcag acaggaggca gtcttggaac ggtgaaaaag cctgc | DNA |
| 39 | primer | gcaggctttt tcaccgttccaagactgcct cctgtctgac tcgtc | DNA |
| 40 | primer | cgctcttgat cttcggatag tggtggtatt gtgactgggg a | DNA |
| 41 | primer | tccccagtca caataccacc actatccgaa gatcaagagc g | DNA |
| 42 | primer | catatgqagt gttatttgaa ggggtcttgg aacggtgaaa aagcctgc | DNA |
| 43 | primer | gcaggctttt tcaccgttcc aagacccctt caaataacac tccatatg | DNA |
| 44 | primer | ccgatacagt gtccaagtac g | DNA |
| 45 | primer | gagtgtctga agacaaggct tc | DNA |
| 46 | primer | gacgacaatg ctgagctccg | DNA |
| 47 | primer | cagctcatct atcttggcct cctgcggtta gtactgcaaa aagtgc | DNA |
| 48 | primer | gcactttttg cagtactaac cgcaggaggc caagatagat gagctg | DNA |
| 49 | primer | cttcgctctt gatcttcgga tagtcacaat tcggataagt ggtctattat atataac | DNA |
| 50 | primer | gttatatata atagaccact tatccgaatt gtgactatcc gaagatcaag agcgaag | DNA |
| 51 | primer | cttgaaatcc gcgctgttat aatcctgcgg ttagtactgc aaaaagtgc | DNA |
| 52 | primer | gcactttttg cagtactaac cgcaggatta taacagcgcg gatttcaag | DNA |
| 53 | primer | cttcgctctt gatcttcgga tagtcactgc gcatcctcaa agtac | DNA |
| 54 | primer | gtactttgag gatgcgcagt gactatccga agatcaagag cgaag | DNA |
| 55 | primer | ggcgtttcag gtggttgcgt gagtg | DNA |
| 56 | primer | gacacaaatg cgccgccaac ccggtctctg cggcggttcg tggttcgtgt ttc | DNA |
| 57 | primer | gaaacacgaa ccacgaaccg ccgcagagac cgggttggcg gcgcatttgt gtc | DNA |
| 58 | primer | cagtcgatca gcatcaggcc ctgcggttag tactgcaaaa | DNA |
| 59 | primer | ttttgcagta ctaaccgcag ggcctgatgc tgatcgactg | DNA |
| 60 | primer | aactaattac atgactcgag ctagttcttg atcttgattc | DNA |
| 61 | primer | gaatcaagat caagaactag ctcgagtcat gtaattagtt | DNA |
| 62 | primer | gacgagtcag acaggaggca gcaaattaaa gccttcgagc gtccc | DNA |
| 63 | primer | gggacgctcg aaggctttaa tttgctgcct cctgtctgac tcgtc | DNA |
| 64 | primer | aactaattac atgactcgag tggtggtatt gtgactgggg | DNA |
| 65 | primer | ccccagtcac aataccacca ctcgagtcat gtaattagtt | DNA |
| 66 | primer | gcagcagtca tacatgttct gaggcaaatt aaagccttcg agcgtccc | DNA |
| 67 | primer | gggacgctcg aaggctttaa tttgcctcag aacatgtatg actgctgc | DNA |
| 68 | primer | ctactttgtg cagattgagg ccaag | DNA |
| 69 | primer | gtcgtctgtc ttctcttcag | DNA |
| 70 | primer | ccaccaagat gggcaagaag | DNA |
| 71 | crtYB | atgacggctc tcgcatatta ccagatccat ctgatctata ctctcccaat tcttggtctt 60 ctcggcctgc tcacttcccc gattttgaca aaatttgaca tctacaaaat atcgatcctc 120 gtatttattg cgtttagtgc aaccacacca tgggactcat ggatcatcag aaatggcgca 180 tggacatatc catcagcgga gagtggccaa ggcgtgtttg gaacgtttct agatgttcca 240 tatgaagagt acgctttctt tgtcattcaa accgtaatca ccggcttggt ctacgtcttg 300 gcaactaggc accttctccc atctctcgcg cttcccaaga ctagatcgtc cgccctttct 360 ctcgcgctca aggcgctcat ccctctgccc attatctacc tatttaccgc tcaccccagc 420 ccatcgcccg acccgctcgt gacagatcac tacttctaca tgcgggcact ctccttactc 480 atcaccccac ctaccatgct cttggcagca ttatcaggcg aatatgcttt cgattggaaa 540 | DNA |
| | | agtggccgag caaagtcaac tattgcagca atcatgatcc cgacggtgta tctgatttgg 600 gtagattatg ttgctgtcgg tcaagactct tggtcgatca acgatgagaa gattgtaggg 660 tggaggcttg gaggtgtact acccattgag gaagctatgt tcttcttact gacgaatcta 720 atgattgttc tgggtctgtc tgcctgcgat catactcagg ccctatacct gctacacggt 780 cgaactattt atggcaacaa aaagatgcca tcttcatttc ccctcattac accgcctgtg 840 ctctccctgt tttttagcag ccgaccatac tcttctcagc caaaacgtga cttggaactg 900 gcagtcaagt tgttggagga aaagagccgg agcttttttg ttgcctcggc tggatttcct 960 | |
| | | agcgaagtta gggagaggct ggttggacta tacgcattct gccgggtgac tgatgatctt 1020 atcgactctc ctgaagtatc ttccaacccg catgccacaa ttgacatggt ctccgatttt 1080 cttaccctac tatttgggcc cccgctacac ccttcgcaac ctgacaagat cctttcttcg 1140 cctttacttc ctccttcgca cccttcccga cccacgggaa tgtatcccct cccgcctcct 1200 ccttcgctct cgcctgccga gctcgttcaa ttccttaccg aaagggttcc cgttcaatac 1260 catttcgcct tcaggttgct cgctaagttg caaggctgga tccctcgata cccactcgac 1320 gaactcctta gaggatacac cactgatctt atctttccct tatcgacaga ggcagtccag 1380 gctcggaaga cgcctatcga gaccacagct gacttgctgg actatggtct atgtgtagca 1440 ggctcagtcg ccgagctatt ggtctatgtc tcttgggcaa gtgcaccaag tcaggtccct 1500 gccaccatag aagaaagaga agctgtgtta gtggcaagcc gagagatggg aactgccctt 1560 cagttggtga acattgctag ggacattaaa ggggacgcaa cagaagggag attttaccta 1620 ccactctcat tctttggtct tcgggatgaa tcaaagcttg cgatcccgac tgattggacg 1680 gaacctcggc ctcaagattt cgacaaactc ctcagtctat ctccttcgtc cacattacca 1740 tcttcaaacg cctcagaaag cttccggttc gaatggaaga cgtactcgct tccattagtc 1800 gcctacgcag aggatcttgc caaacattct tataagggaa ttgaccgact tcctaccgag 1860 gttcaagcgg gaatgcgagc ggcttgcgcg agctacctac tgatcggccg agagatcaaa 1920 gtcgtttgga aaggagacgt cggagagaga aggacagttg ccggatggag gagagtacgg 1980 aaagtcttga gtgtggtcat gagcggatgg gaagggcagt aa | |
| 72 | crtl | atgggaaaag aacaagatca ggataaaccc acagctatca tcgtgggatg tggtatcggt 60 ggaatcgcca ctgccgctcg tcttgctaaa gaaggtttcc aggtcacggt gttcgagaag 120 aacgactact ccggaggtcg atgctcttta atcgagcgag atggttatcg attcgatcag 180 gggcccagtt tgctgctctt gccagatctc ttcaagcaga cattcgaaga tttgggagag 240 aagatggaag attgggtcga tctcatcaag tgtgaaccca actatgtttg ccacttccac 300 gatgaagaga ctttcactct ttcaaccgac atggcgttgc tcaagcggga agtcgagcgt 360 tttgaaggca aagatggatt tgatcggttc ttgtcgttta tccaagaagc ccacagacat 420 tacgagcttg ctgtcgttca cgtcctgcag aagaacttcc ctggcttcgc agcattctta 480 cggctacagt tcattggcca aatcctggct cttcacccct tcgagtctat ctggacaaga 540 gtttgtcgat atttcaagac cgacagatta cgaagagtct tctcgtttgc agtgatgtac 600 | DNA |
| | | atgggtcaaa gcccatacag tgcgcccgga acatattcct tgctccaata caccgaattg 660 accgagggca tctggtatcc gagaggaggc ttttggcagg ttcctaatac tcttcttcag 720 atcgtcaagc gcaacaatcc ctcagccaag ttcaatttca acgctccagt ttcccaggtt 780 cttctctctc ctgccaagga ccgagcgact ggtgttcgac ttgaatccgg cgaggaacat 840 cacgccgatg ttgtgattgt caatgctgac ctcgtttacg cctccgagca cttgattcct 900 gacgatgcca gaaacaagat tggccaactg ggtgaagtca agagaagttg gtgggctgac 960 ttagttggtg gaaagaagct caagggaagt tgcagtagtt tgagcttcta ctggagcatg 1020 gaccgaatcg tggacggtct gggcggacac aatatcttct tggccgagga cttcaaggga 1080 tcattcgaca caatcttcga ggagttgggt ctcccagccg atccttcctt ttacgtgaac 1140 gttccctcgc gaatcgatcc ttctgccgct cccgaaggca aagatgctat cgtcattctt 1200 gtgccgtgtg gccatatcga cgcttcgaac cctcaagatt acaacaagct tgttgctcgg 1260 gcaaggaagt ttgtgatcca cacgctttcc gccaagcttg gacttcccga ctttgaaaaa 1320 atgattgtgg cagagaaggt tcacgatgct ccctcttggg agaaagaatt caacctcaag 1380 gacggaagca tcttgggact ggctcacaac tttatgcaag ttcttggttt caggccgagc 1440 accagacatc ccaagtatga caagttgttc tttgtcgggg cttcgactca tcccggaact 1500 | |
| | | ggggttccca tcgtcttggc tggagccaag ttaactgcca accaagttct cgaatccttt 1560 gaccgatccc cagctccaga tcccaatatg tcactctccg taccatatgg aaaacctctc 1620 aaatcaaatg gaacgggtat cgattctcag gtccagctga agttcatgga tttggagaga 1680 tgggtatacc ttttggtgtt gttgattggg gccgtgatcg ctcgatccgt tggtgttctt 1740 gctttctga | |
| 73 | TEFINtp-crtYB-CYC1t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 | DNA |
| | | acacaagacg ccacgtcatc cgaccagcac tttttgcagt actaaccgca gacggctctc 540 gcatattacc agatccatct gatctatact ctcccaattc ttggtcttct cggtctgctc 600 acttccccga ttttgacaaa atttgacatc tacaaaatat cgatcctcgt atttattgcg 660 tttagtgcaa ccacaccatg ggactcatgg atcatcagaa atggcgcatg gacatatcca 720 tcagcggaga gtggccaagg cgtgtttgga acgtttctag atgttccata tgaagagtac 780 gctttctttg tcattcaaac cgtaatcacc ggcttggtct acgtcttggc aactaggcac 840 cttctcccat ctctcgcgct tcccaagact agatcgtccg ccctttctct cgcgctcaag 900 gcgctcatcc ctctgcccat tatctaccta tttaccgctc accccagccc atcgcccgac 960 ccgctcgtga cagatcacta cttctacatg cgggcactct ccttactcat caccccacct 1020 accatgctct tggcagcatt atcaggcgaa tatgctttcg attggaaaag tggccgagca 1080 aagtcaacta ttgcagcaat catgatcccg acggtgtatc tgatttgggt agattatgtt 1140 gctgtcggtc aagactcttg gtcgatcaac gatgagaaga ttgtagggtg gaggcttgga 1200 ggtgtactac ccattgagga agctatgttc ttcttactga cgaatctaat gattgttctg 1260 ggtctgtctg cctgcgatca tactcaggcc ctatacctgc tacacggtcg aactatttat 1320 | |
| | | ggcaacaaaa agatgccatc ttcatttccc ctcattacac cgcctgtgct ctccctgttt 1380 tttagcagcc gaccatactc ttctcagcca aaacgtgact tggaactggc agtcaagttg 1440 ttggaggaaa agagccggag cttttttgtt gcctcggctg gatttcctag cgaagttagg 1500 gagaggctgg ttggactata cgcattctgc cgggtgactg atgatcttat cgactctcct 1560 gaagtatctt ccaacccgca tgccacaatt gacatggtct ccgattttct taccctacta 1620 tttgggcccc cgctacaccc ttcgcaacct gacaagatcc tttcttcgcc tttacttcct 1680 ccttcgcacc cttcccgacc cacgggaatg tatcccctcc cgcctcctcc ttcgctctcg 1740 cctgccgagc tcgttcaatt ccttaccgaa agggttcccg ttcaatacca tttcgccttc 1800 aggttgctcg ctaagttgca agggctgatc cctcgatacc cactcgacga actccttaga 1860 ggatacacca ctgatcttat ctttccttta tcgacagagg cagtccaggc tcggaagacg 1920 cctatcgaga ccacagctga cttgctggac tatggtctat gtgtagcagg ctcagtcgcc 1980 gagctattgg tctatgtctc ttgggcaagt gcaccaagtc aggtccctgc caccatagaa 2040 gaaagagaag ctgtgttagt ggcaagccga gagatgggaa ctgcccttca gttggtgaac 2100 attgctaggg acattaaagg ggacgcaaca gaagggagat | |
| | | tttacctacc actctcattc 2160 tttggtcttc gggatgaatc aaagcttgcg atcccgactg attggacgga acctcggcct 2220 caagatttcg acaaactcct cagtctatct ccttcgtcca cattaccatc ttcaaacgcc 2280 tcagaaagct tccggttcga atggaagacg tactcgcttc cattagtcgc ctacgcagag 2340 gatcttgcca aacattctta taagggaatt gaccgacttc ctaccgaggt tcaagcggga 2400 atgcgagcgg cttgcgcgag ctacctactg atcggccgag agatcaaagt cgtttggaaa 2460 ggagacgtcg gagagagaag gacagttgcc ggatggagga gagtacggaa agtcttgagt 2520 gtggtcatga gcggatggga agggcagtaa ctcgagtcat gtaattagtt atgtcacgct 2580 tacattcacg ccctcccccc acatccgctc taaccgaaaa ggaaggagtt agacaacctg 2640 aagtctaggt ccctatttat ttttttatag ttatgttagt attaagaacg ttatttatat 2700 ttcaaatttt tctttttttt ctgtacagac gcgtgtacgc atgtaacatt atactgaaaa 2760 ccttgcttga gaaggttttg ggacgctcga aggctttaat ttgc | |
| 74 | TEFINtp-crtl-CYC1t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca gggaaaagaa 540 caagatcagg ataaacccac agctatcatc gtgggatgtg gtatcggtgg aatcgccact 600 gccgctcgtc ttgctaaaga aggtttccag gtcacggtgt tcgagaagaa cgactactcc 660 ggaggtcgat gctctttaat cgagcgagat ggttatcgat tcgatcaggg gcccagtttg 720 ctgctcttgc cagatctctt caagcagaca ttcgaagatt tgggagagaa gatggaagat 780 tgggtcgatc tcatcaagtg tgaacccaac tatgtttgcc acttccacga tgaagagact 840 ttcactcttt caaccgacat ggcgttgctc aagcgggaag tcgagcgttt tgaaggcaaa 900 gatggatttg atcggttctt gtcgtttatc caagaagccc acagacatta cgagcttgct 960 gtcgttcacg tcctgcagaa gaacttccct ggcttcgcag cattcttacg | DNA |
| | | gctacagttc 1020 attggccaaa tcctggctct tcaccccttc gagtctatct ggacaagagt ttgtcgatat 1080 ttcaagaccg acagattacg aagagtcttc tcgtttgcag tgatgtacat gggtcaaagc 1140 ccatacagtg cgcccggaac atattccttg ctccaataca ccgaattgac cgagggcatc 1200 tggtatccga gaggaggctt ttggcaggtt cctaatactc ttcttcagat cgtcaagcgc 1260 aacaatccct cagccaagtt caatttcaac gctccagttt cccaggttct tctctctcct 1320 gccaaggacc gagcgactgg tgttcgactt gaatccggcg aggaacatca cgccgatgtt 1380 gtgattgtca atgctgacct cgtttacgcc tccgagcact tgattcctga cgatgccaga 1440 aacaagattg gccaactggg tgaagtcaag agaagttggt gggctgactt agttggtgga 1500 aagaagctca agggaagttg cagtagtttg agcttctact ggagcatgga ccgaatcgtg 1560 | |
| | | gacggtctgg gcggacacaa tatcttcttg gccgaggact tcaagggatc attcgacaca 1620 atcttcgagg agttgggtct cccagccgat ccttcctttt acgtgaacgt tccctcgcga 1680 atcgatcctt ctgccgctcc cgaaggcaaa gatgctatcg tcattcttgt gccgtgtggc 1740 catatcgacg cttcgaaccc tcaagattac aacaagcttg ttgctcgggc aaggaagttt 1800 gtgatccaca cgctttccgc caagcttgga cttcccgact ttgaaaaaat gattgtggca 1860 gagaaggttc acgatgctcc ctcttgggag aaagaattca acctcaagga cggaagcatc 1920 ttgggactgg ctcacaactt tatgcaagtt cttggtttca ggccgagcac cagacatccc 1980 aagtatgaca agttgttctt tgtcggggct tcgactcatc ccggaactgg ggttcccatc 2040 gtcttggctg gagccaagtt aactgccaac caagttctcg aatcctttga ccgatcccca 2100 gctccagatc ccaatatgtc actctccgta ccatatggaa aacctctcaa atcaaatgga 2160 acgggtatcg attctcaggt ccagctgaag ttcatggatt tggagagatg ggtatacctt 2220 ttggtattgt tgattggggc cgtgatcgct cgatccgttg gtgttcttgc tttctgactc 2280 gagtcatgta attagttatg tcacgcttac attcacgccc tccccccaca tccgctctaa 2340 ccgaaaagga aggagttaga caacctgaag tctaggtccc tatttatttt tttatagtta 2400 tgttagtatt aagaacgtta tttatatttc aaatttttct tttttttctg tacagacgcg 2460 tgtacgcatg taacattata ctgaaaacct tgcttgagaa ggttttggga cgctcgaagg 2520 ctttaatttg c | |
| 75 | URA3 | tgcctcctgt ctgactcgtc attgccgcct ttggagtacg actccaacta tgagtgtgct 60 tggatcactt tgacgataca ttcttcgttg gaggctgtgg gtctgacagc | DNA |
| | | tgcgttttcg 120 gcgcggttgg ccgacaacaa tatcagctgc aacgtcattg ctggctttca tcatgatcac 180 atttttgtcg gcaaaggcga cgcccagaga gccattgacg ttctttctaa tttggaccga 240 tagccgtata gtccagtcta tctataagtt caactaactc gtaactatta ccataacata 300 tacttcactg ccccagataa ggttccgata aaaagttctg cagactaaat ttatttcagt 360 ctcctcttca ccaccaaaat gccctcctac gaagctcgag ctaacgtcca caagtccgcc 420 tttgccgctc gagtgctcaa gctcgtggca gccaagaaaa ccaacctgtg tgcttctctg 480 gatgttacca ccaccaagga gctcattgag cttgccgata aggtcggacc ttatgtgtgc 540 atgatcaaga cccatatcga catcattgac gacttcacct acgccggcac tgtgctcccc 600 ctcaaggaac ttgctcttaa gcacggtttc ttcctgttcg aggacagaaa gttcgcagat 660 attggcaaca ctgtcaagca ccagtacaag aacggtgtct accgaatcgc cgagtggtcc 720 gatatcacca acgcccacgg tgtacccgga accggaatca ttgctggcct gcgagctggt 780 gccgaggaaa ctgtctctga acagaagaag gaggacgtct ctgactacga gaactcccag 840 tacaaggagt tcctggtccc ctctcccaac gagaagctgg ccagaggtct gctcatgctg 900 gccgagctgt cttgcaaggg ctctctggcc actggcgagt actccaagca gaccattgag 960 cttgcccgat ccgaccccga gtttgtggtt ggcttcattg cccagaaccg acctaagggc 1020 gactctgagg actggcttat tctgaccccc ggggtgggtc ttgacgacaa gggagacgct 1080 ctcggacagc agtaccgaac tgttgaggat gtcatgtcta ccggaacgga tatcataatt 1140 gtcggccgag gtctgtacgg ccagaaccga gatcctattg aggaggccaa gcgataccag 1200 aaggctggct gggaggctta ccagaagatt aactgttaga ggttagacta tggatatgtc 1260 atttaactgt gtatatagag agcgtgcaag tatggagcgc ttgttcagct tgtatgatgg 1320 tcagacgacc tgtctgatcg agtatgtatg atactgcaca acctgtgtat ccgcatgatc 1380 tgtccaatgg ggcatgttgt tgtgtttctc gatacggaga tgctgggtac aagtagctaa 1440 tacgattgaa ctacttatac ttatatgagg cttgaagaaa gctgacttgt gtatgactta 1500 ttctcaacta catccccagt cacaatacca cca | |
| 76 | primer | gtgcgcttct ctcgtctcgg taaccctgtc | DNA |
| 77 | primer | atgcgccgcc aacccggtct ctggggtgtg gtggatgggg tgtg | DNA |
| 78 | primer | cacaccccat ccaccacacc ccagagaccg ggttggcggc gcat | DNA |
| 79 | primer | cgccqccaac ccggtctctt gaagacgaaa gggcctccg | DNA |
| 80 | primer | cggaggccct ttcgtcttca agagaccggg ttggcggcg | DNA |
| 81 | primer | gacgagtcag acaggaggca tcagacagat actcgtcgcg | DNA |
| 82 | primer | cgcgacgagt atctgtctga tgcctcctgt ctgactcgtc | DNA |
| 83 | primer | atgacqagtc agacaggagg catggtggta ttgtgactgg ggat | DNA |
| 84 | primer | atccccagtc acaataccac catqcctcct gtctgactcg teat | DNA |
| 85 | primer | cgqcgtcctt ctcgtagtcc gcttttggtg gtgaagagga gact | DNA |
| 86 | primer | agtctcctct tcaccaccaa aagcggacta cgagaaggac gccg | DNA |
| 87 | primer | ccactcgtca ccaacaqtgc cgtgtgttgc | DNA |
| 88 | primer | tcgtacqtct ataccaacag atgg | DNA |
| 89 | primer | cgcatacaca cacactgccg gggg | DNA |
| 90 | HMGR native promoter | tccacacgtc gttctttttt ccttagcctt ttttgcagtg cgcgtgtccc aaaccccagc 60 tctacacacc agcacaaaca aagttaagct cagggttgtc gttgaggtcg cttactgtag 120 tcagtgctcg tatggttcgt tcaattttcg ccaaaaatcg ttttgccttt gtatcttggg 180 aataacatca actgtggttc ttcaacaggc ctaaggaacg aaacaagccg gaccaagatc 240 aggttcaagg tgagtactga gaaggaatag aaggcctaaa ggcgcaaacc gacaggtggc 300 aacagctcca caccgaccac gaaggccacg aaatcaaggg gtcctaaagt tagtctttgt 360 ggcctcgacg gtcagcgaaa acgcgagacc acaacgcgat cagaaccagg acctaaacaa 420 cacaggacgg ggtcacaata ggcttgaaca gcaagtacaa gctgtgatct ctctatattt 480 gattctcaaa ccacccctga ctacttcagc gcctctgtga cacagccccc ctatcatccg 540 actaacacag | DNA |
| 91 | primer | gacaatqcct cgaggaggtt taaaagtaac t | DNA |
| 92 | primer | gcgccgccaa cccggtctct ctgtgttagt cggatgatag g | DNA |
| 93 | primer | cctatcatcc gactaacaca gagagaccgg gttggcggcg c | DNA |
| 94 | primer | gacqagtcag acaggaggca ctgcggttag tactgcaaaa ag | DNA |
| 95 | primer | ctttttgcaq tactaaccgc agtgcctcct gtctgactcg tc | DNA |
| 96 | primer | atgcgccgcc aacccggtct cttggtggta ttgtgactgg ggat | DNA |
| 97 | primer | atccccagtc acaataccac caagagaccg ggttggcggc gcat | DNA |
| 98 | primer | ctttccaata gctgcttgta gctgcggtta gtactgcaaa a | DNA |
| 99 | primer | ttttgcaqta ctaaccgcag ctacaagcag ctattggaaa g | DNA |
| 100 | primer | gcttaatgtg attgatctca aacttgatag | DNA |
| 101 | primer | gctgtctctg cgagagcacg tcga | DNA |
| 102 | primer | gqttcqcaca acttctcggg tggc | DNA |
| 103 | Hp.GGPP | MIRAMHNRAP TPRTRVSHPR SHRALAHVSA VATAGQVAEV HSAPAFDFEM YMRDRAEMVN 60 KALDAALPSR YPEVLVDSMR YSVLAGGKRV RPALTLAACD LVGGDMATAL PTACAMEMIH 120 TMSLIHDDLP AMDNDDFRRG RPTNHKVYGE DIAILAGDAL LSFAFEHIAR DTKGVPADAV 180 LKVIMELGRA VGAQGLSAGQ AVDIKSEGQE VGLEVLEYIH HHKTAALLEA AVVCGALVGG 240 ADTATVEKLR KYALNIGLAF QVIDDILDVT QTTETLGKTA AKDLAVNKTT YPKLLGLEAS 300 RKVADDLIRE AIAQLDEFEP ARKAPMVALA HLIGYRKN | Protein |

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-0125 | Accession number given by the
INTERNATIONAL DEPOSITARY AUTHORITY:
KCCM12972P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 09. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 09. 2021.

¹ Where Rule 6.4(d) applies, such date is the date on which the status of international depositary authority was
acquired.

Form BP/4 (sole page)
한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-0125 | Accession number given by the
INTERNATIONAL DEPOSITARY AUTHORITY:
KCCM12972P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 09. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 09. 2021.

¹ Where Rule 6.4(d) applies, such date is the date on which the status of international depositary authority was
acquired.

Form BP/4 (sole page)
한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. A microorganism of the genus *Yarrowia* having an ability to produce carotenoid or a material having carotenoid as a precursor, the microorganism expressing *Haematococcus pluvialis-derived* geranylgeranyl pyrophosphate synthase.

2. The microorganism of claim 1, wherein the geranylgeranyl pyrophosphate synthase consists of an amino acid sequence of SEQ ID NO: 103.

3. The microorganism of claim 1, wherein the geranylgeranyl pyrophosphate synthase is encoded by a polynucleotide consisting of a nucleotide sequence of SEQ ID NO: 1.

4. The microorganism of claim 1, wherein the microorganism of the genus *Yarrowia* is *Yarrowia lipolytica.*

5. The microorganism of claim 1, wherein the material having carotenoid as a precursor is retinoid.

6. The microorganism of claim 1, wherein the carotenoid is beta-carotene.

7. The microorganism of claim 5, wherein the retinoid is retinol.

8. The microorganism of claim 1, wherein the microorganism of the genus *Yarrowia* has a reduced ability to produce a by-product.

9. The microorganism of claim 8, wherein the by-product is squalene.

10. A method of producing carotenoid or a material having carotenoid as a precursor, the method comprising the steps of:
culturing the microorganism of the genus *Yarrowia* of any one of claims 1 to 9 in a medium; and
recovering carotenoid or the material having carotenoid as a precursor from the microorganism of the genus *Yarrowia* or the medium.

11. The method of claim 10, further comprising the step of:
converting beta-carotene, which is produced by the microorganism of the genus *Yarrowia,* into carotenoids other than beta-carotene; or
converting retinol, which is produced by the microorganism of the genus *Yarrowia,* into retinoids other than retinol.

12. A composition for producing carotenoid or a material having carotenoid as a precursor, the composition comprising the microorganism of the genus *Yarrowia* of any one of claims 1 to 9, or a culture thereof.

13. Use of a microorganism of the genus *Yarrowia* in producing carotenoid or a material having carotenoid as a precursor, the microorganism having an ability to produce carotenoid or a material having carotenoid as a precursor by expressing *Haematococcus pluvialis-derived* geranylgeranyl pyrophosphate synthase.
